# EUROPÄISCHE PATENTSCHRIFT

(11) **EP 1 660 125 B1**
(45) Veröffentlichungstag und Bekanntmachung des Hinweises auf die Patenterteilung: **06.05.2015**
(21) Anmeldenummer: 04764564.3
(22) Anmeldetag: 27.08.2004
(51) Int. Cl.: A61K 39/12, A61K 39/29, C07K 14/02, C07K 14/005, C12N 15/85

(54) **ZUSAMMENSETZUNG ZUR PROPHYLAXE/THERAPIE VON HBV-INFEKTIONEN UND HBV-VERMITTELTEN ERKRANKUNGEN**
COMPOSITION FOR THE PROPHYLAXIS/TREATMENT OF HBV INFECTIONS AND HBV-MEDIATED DISEASES
COMPOSITION SERVANT A PREVENIR/TRAITER DES INFECTIONS PAR LE HBV ET DES MALADIES DONT LA MEDIATION EST ASSUREE PAR LE HBV

(30) Priorität: 29.08.2003 DE 10339927
(43) Veröffentlichungstag der Anmeldung: 31.05.2006
(73) Patentinhaber: Rhein Biotech Gesellschaft für neue biotechnologische Prozesse und Produkte mbH, 40595 Düsseldorf (DE)
(72) Erfinder: MELBER, Karl, 40951 Düsseldorf (DE)
(74) Vertreter: Herzog, Martin
(86) Internationale Anmeldenummer: PCT/EP2004/009590
(87) Internationale Veröffentlichungsnummer: WO 2005/023297

(56) Entgegenhaltungen:
- EP-A- 0 389 983
- EP-A- 0 456 215
- EP-A- 0 511 854
- EP-A- 0 533 263
- WO-A-97/40193
- RU-C- 2 233 672
- RU-C- 2 233 673
- PATENT ABSTRACTS OF JAPAN Bd. 2002, Nr. 02, 2. April 2002 (2002-04-02) & JP 2001 294599 A (SEKISUI CHEM CO LTD), 23. Oktober 2001 (2001-10-23)
- MAGNIUS L O ET AL: "SUBTYPES, GENOTYPES AND MOLECULAR EPIDEMIOLOGY OF THE HEPATITIS B VIRUS AS REFLECTED BY SEQUENCE VARIABILITY OF THE S-GENE.ATITIS B" INTERVIROLOGY, XX, XX, Bd. 38, Nr. 1/2, 1995, Seiten 24-34, XP000197124 ISSN: 0300-5526
- DAVIS H L ET AL: "DNA-based immunization against hepatitis B surface antigen (HBsAg) in normal and HBsAg-transgenic mice" VACCINE, BUTTERWORTH SCIENTIFIC. GUILDFORD, GB, Bd. 15, Nr. 8, Juni 1997 (1997-06), Seiten 849-852, XP004075668 ISSN: 0264-410X
- MANCINI MARYLINE ET AL: "Induction of anti-hepatitis B surface antigen (HBsAg) antibodies in HBsAg producing transgenic mice: A possible way of circumventing "nonresponse" to HBsAg" JOURNAL OF MEDICAL VIROLOGY, Bd. 39, Nr. 1, 1993, Seiten 67-74, XP009040798 ISSN: 0146-6615
- SCHIRMBECK REINHOLD ET AL: "Breaking tolerance in hepatitis B surface antigen (HBsAg) transgenic mice by vaccination with cross-reactive, natural HBsAg variants." EUROPEAN JOURNAL OF IMMUNOLOGY, Bd. 33, Nr. 12, Dezember 2003 (2003-12), Seiten 3342-3352, XP009040725 ISSN: 0014-2980
- SCHIRMBECK REINHOLD ET AL: "Different immunogenicity of H-2 Kb-restricted epitopes in natural variants of the hepatitis B surface antigen." EUROPEAN JOURNAL OF IMMUNOLOGY, Bd. 33, Nr. 9, September 2003 (2003-09), Seiten 2429-2438, XP009040727 ISSN: 0014-2980 in der Anmeldung erwähnt
- SCHIRMBECK R. ET AL: "Enhanced priming od multispecific, murine CD8+ T cell responses by DNA vaccines expressing stress protein-binding polytope peptides", THE JOURNAL OF IMMUNOLOGY, vol. 171, 2003, pages 1240-1246, XP004764564,
- KEDL R.M. ET AL: "Epitope dominance, competition and T cell affinity maturation", CURRENT OPINION IN IMMUNOLOGY, vol. 15, 2003, pages 120-127, XP004764564,
- DESMOND C P ET AL: "A systematic review of T-cell epitopes in hepatitis B virus: Identification, genotypic variation and relevance to antiviral therapeutics", ANTIVIRAL THERAPY- AN OFFICIAL PUBLICATION OF THE INTERNATIONAL SOCIETY FOR ANTIVIRAL RESEARCH, MTM PUBLICATIONS, LONDON, GB, vol. 13, no. 2, 1 January 2008 (2008-01-01), pages 161-175, XP009142238, ISSN: 1359-6535
- DATABASE BIOSIS [Online] BIOSCIENCES INFORMATION SERVICE, PHILADELPHIA, PA, US 1989 MIMMS L ET AL: 'SECOND GENERATION ASSAYS FOR THE DETECTION OF ANTIBODY TO HBSAG USING RECOMBINANT DNA-DERIVED HBSAG' Database accession no. PREV198988096484 & MIMMS L ET AL: "SECOND GENERATION ASSAYS FOR THE DETECTION OF ANTIBODY TO HBSAG USING RECOMBINANT DNA-DERIVED HBSAG", JOURNAL OF VIROLOGICAL METHODS, vol. 25, no. 2, 1989, pages 211-232, ISSN: 0166-0934
- DATABASE BIOSIS [Online] BIOSCIENCES INFORMATION SERVICE, PHILADELPHIA, PA, US August 2007 KAY ALAN ET AL: 'Hepatitis B virus genetic variability and evolution' Database accession no. PREV200700518393 & VIRUS RESEARCH, vol. 127, no. 2, August 2007 (2007-08), pages 164-176, ISSN: 0168-1702, DOI: 10.1016/J.VIRUSRES.2007.02.021
- DATABASE BIOSIS [Online] BIOSCIENCES INFORMATION SERVICE, PHILADELPHIA, PA, US März 2001 PAGE M ET AL: 'A novel, recombinant triple antigen hepatitis B vaccine (hepacare(R))' Database accession no. PREV200100475422 & PAGE M ET AL: "A novel, recombinant triple antigen hepatitis B vaccine (hepacare(R))", INTERVIROLOGY, vol. 44, no. 2-3, March 2001 (2001-03), pages 88-97, ISSN: 0300-5526
- DATABASE BIOSIS [Online] BIOSCIENCES INFORMATION SERVICE, PHILADELPHIA, PA, US Februar 2000 SOBOTTA DIRK ET AL: 'Mapping of immunodominant B-cell epitopes and the human serum albumin-binding site in natural hepatitis B virus surface antigen of defined genosubtype' Database accession no. PREV200000139327 & SOBOTTA DIRK ET AL: "Mapping of immunodominant B-cell epitopes and the human serum albumin-binding site in natural hepatitis B virus surface antigen of defined genosubtype", JOURNAL OF GENERAL VIROLOGY, vol. 81, no. 2, February 2000 (2000-02), pages 369-378, ISSN: 0022-1317
- J. KNESER & H. WIEDEMEYER: "Die Bedeutung der HBV-Genotypen bei chronischer HBV-HBV/HIV-Infektion", HIV & MORE, February 2005 (2005-02),

### SEQUENZPROTOKOLL

<110> Rhein Biotech Gesellschaft für neue biotechnologische Prozesse und
   Produkte mbH Melber, Karl
<120> Zusammensetzung zur Prophylaxe/Therapie von HBV-Infektionen und HBV-vermittelten Erkrankungen
<130> PCT-2069HVTF
<140>
   <141>
<150> DE10339927.5
   <151> 2003-08-29
<160> 7
<170> PatentIn Ver. 2.1
<210> 1
   <211> 226
   <212> PRT
   <213> Hepatitis B virus
<220>
   <223> HBsAg-Polypeptid von HBV Subtyp adw2/Genotyp A
<400> 1
   Met Glu Asn Ile Thr Ser Gly Phe Leu Gly Pro Leu Leu Val Leu Gln
    1               5                   10                  15
   Ala Gly Phe Phe Leu Leu Thr Arg Ile Leu Thr Ile Pro Gln Ser Leu
               20                  25                  30
   Asp Ser Trp Trp Thr Ser Leu Asn Phe Leu Gly Gly Ser Pro Val Cys
           35                  40                  45
   Leu Gly Gln Asn Ser Gln Ser Pro Thr Ser Asn His Ser Pro Thr Ser
       50                  55                  60
   Cys Pro Pro Ile Cys Pro Gly Tyr Arg Trp Met Cys Leu Arg Arg Phe
   65                  70                  75                  80
   Ile Ile Phe Leu Phe Ile Leu Leu Leu Cys Leu Ile Phe Leu Leu Val
                   85                  90                  95
   Leu Leu Asp Tyr Gln Gly Met Leu Pro Val Cys Pro Leu Ile Pro Gly
               100                 105                 110
   Ser Thr Thr Thr Ser Thr Gly Pro Cys Lys Thr Cys Thr Thr Pro Ala
           115                 120                 125
   Gln Gly Asn Ser Met Phe Pro Ser Cys Cys Cys Thr Lys Pro Thr Asp
       130                 135                 140
   Gly Asn Cys Thr Cys Ile Pro Ile Pro Ser Ser Trp Ala Phe Ala Lys
   145                 150                 155                 160
   Tyr Leu Trp Glu Trp Ala Ser Val Arg Phe Ser Trp Leu Ser Leu Leu
                   165                 170                 175
   Val Pro Phe Val Gln Trp Phe Val Gly Leu Ser Pro Thr Val Trp Leu
               180                 185                 190
   Ser Ala Ile Trp Met Met Trp Tyr Trp Gly Pro Ser Leu Tyr Ser Ile
           195                 200                 205
   Val Ser Pro Phe Ile Pro Leu Leu Pro Ile Phe Phe Cys Leu Trp Val
       210                 215                 220
   Tyr Ile
   225
<210> 2
   <211> 226
   <212> PRT
   <213> Hepatitis B virus
<220>
   <223> HBsAg-Polypeptid von HBV Subtyp ayw/Genotyp D
<400> 2
   Met Glu Asn Ile Thr Ser Gly Phe Leu Gly Pro Leu Leu Val Leu Gln
   1 5 10 15
   Ala Gly Phe Phe Leu Leu Thr Arg Ile Leu Thr Ile Pro Gln Ser Leu
   20 25 30
   Asp Ser Trp Trp Thr Ser Leu Asn Phe Leu Gly Gly Thr Thr Val Cys
   35 40 45
   Leu Gly Gln Asn Ser Gln Ser Pro Thr Ser Asn His Ser Pro Thr Ser
   50 55 60
   Cys Pro Pro Thr Cys Pro Gly Tyr Arg Trp Met Cys Leu Arg Arg Phe
   65 70 75 80
   Ile Ile Phe Leu Phe Ile Leu Leu Leu Cys Leu Ile Phe Leu Leu Val
   85 90 95
   Leu Leu Asp Tyr Gln Gly Met Leu Pro Val Cys Pro Leu Ile Pro Gly
   100 105 110
   Ser Ser Thr Thr Ser Thr Gly Pro Cys Arg Thr Cys Met Thr Thr Ala
   115 120 125
   Gln Gly Thr Ser Met Tyr Pro Ser Cys Cys Cys Thr Lys Pro Ser Asp
   130 135 140
   Gly Asn Cys Thr Cys Ile Pro Ile Pro Ser Ser Trp Ala Phe Gly Lys
   145 150 155 160
   Phe Leu Trp Glu Trp Ala Ser Ala Arg Phe Ser Trp Leu Ser Leu Leu
   165 170 175
   Val Pro Phe Val Gln Trp Phe Val Gly Leu Ser Pro Thr Val Trp Leu
   180 185 190
   Ser Val Ile Trp Met Met Trp Tyr Trp Gly Pro Ser Leu Tyr Ser Ile
   195 200 205
   Leu Ser Pro Phe Leu Pro Leu Leu Pro Ile Phe Phe Cys Leu Trp Val
   210 215 220
   Tyr Ile
   225
<210> 3
   <211> 8
   <212> PRT
   <213> Hepatitis B virus
<220>
   <223> HBV Subtyp adw2 HBsAg Epitop 190-197
<400> 3
   Val Trp Leu Ser Ala Ile Trp Met
   1 5
<210> 4
   <211> 8
   <212> PRT
   <213> Hepatitis B virus
<220>
   <223> HBV Subtyp ayw HBsAg Epitop 190-197
<400> 4
   Val Trp Leu Ser Val Ile Trp Met
   1               5
<210> 5
   <211> 8
   <212> PRT
   <213> Hepatitis B virus
<220>
   <223> HBV Subtyp adw2 HBsAg Epitop 208-215
<400> 5
   Ile Val Ser Pro Phe Ile Pro Leu
   1               5
<210> 6
   <211> 8
   <212> PRT
   <213> Hepatitis B virus
<220>
   <223> HBV Subtyp ayw HBsAg Epitop 208-215
<400> 6
   Ile Leu Ser Pro Phe Leu Pro Leu
   1               5
<210> 7
   <211> 225
   <212> PRT
   <213> Hepatitis B virus
<220>
   <223> HBsAg-Polypeptid von HBV Subtyp adr/Genotyp C
<400> 7

## Beschreibung

Die vorliegende Erfindung betrifft Zusammensetzungen, die wenigstens zwei Hepatitis B Virus Oberflächen-Antigene (HBsAg's), Fragmente davon und/oder diese kodierende Nukleinsäuren enthalten, wobei sich die HBsAg's im Hepatitis B Virus (HBV) Genotyp in der S-Region und/oder Prä-S1-Region von HBsAg unterscheiden, wobei die Zusammensetzung kein HBV Core Antigen (HBcAg) oder dieses kodierende Nukleinsäure enthält: pharmazeutische Zusammensetzungen, insbesondere Vakzine enthaltend diese Zusammensetzungen und deren Verwendung zur Prophylaxe/Therapie einer HBV-Infektion oder einer durch HBV-vermittelten Erkrankung. Die vorliegende Erfindung betrifft ferner ein Verfahren zur Herstellung eines Patienten-spezifischen Arzneimittels zur therapeutischen Behandlung von Hepatitis.

Mehr als 250 Millionen Menschen sind weltweit mit Hepatitis B Virus (HBV) infiziert. Ein bedeutender Teil der Infizierten zeigt pathologische Konsequenzen, einschließlich chronischer hepatischer Insuffizienz, Zirrhose und hepatozellulärem Karzinom (HCC). Die Ursache, warum bestimmte Personen eine akute HBV-Infektion entwickeln, während andere dies nicht tun, ist wenig verstanden. Klinische Daten und die Analogie mit weiteren chronischen viralen Infektionen haben die Bedeutung einer Zell-vermittelten Immunantwort bei der Bekämpfung viraler Infektionen betont, insbesondere einer solchen Immunantwort, die zytotoxische T-Lymphozyten umfasst. Die Induktion einer zytotoxischen T-Zellantwort ist ein kritischer Faktor, die akute HBV-Infektion zu beseitigen und die chronische HBV-Infektion zu verhindern. Das virale Genom kodiert unter anderem die Envelope-Proteine Prä-S1, Prä-S2 und das S-Antigen (HBsAg), die Polymerase und das Core-Protein (HBcAg).

Die chronische Hepatitis B stellt eine progredient verlaufende Leberentzündung dar, die chronisch persistierend oder chronisch aggressiv verlaufen kann. Die chronisch persistierende Hepatitis zeigt eine auf die verbreiteten Portatfelder der Leber beschränkte Infiltration mit zunehmender Fibrosierung; klinisch bestehen jahrelang (bis zu 10 Jahren) Zeichen der persistierenden Hepatitis, ca. 80 % der Fälle sind HBsAg-positiv. Die Pathogenese beruht wahrscheinlich auf der Insuffizienz des zellulären Immunsystems und persistierender Virusinfektion.

Das kleine Hepatitis B Oberflächen-Antigen (HBsAg), ein 226-Aminosäuren Protein (p24/gp27 oder S-Protein), ist ein prominentes HBV-Antigen, das in 20-30 nm Lipoproteinpartikel selbst assembliert, in denen 100-150 Untereinheiten durch multiple inter- und intra-molekulare Disulfidbindungen quervernetzt sind. Die Variabilität des S Proteins aus HBV-Isolaten unterschiedlicher Subtypen und Genotypen ist begrenzt. Die vier stabilen, HBsAg Subtypen adw, ayw, adr und ayr betreffen einzelne Aminosäureaustausche an den Positionen 122 und 160, die benachbart zur immunodominanten "a-Determinante" (eine hydrophile Region, die die Reste 124-147 umfasst) gelegen sind. Bisher wurden diesen Subtypen keine biologischen oder pathogenetischen Unterschiede bei der HBV-Infektion zugeordnet.

1982 wurde in der Bundesrepublik Deutschland erstmals ein Impfstoff (Vakzin) zugelassen, der aus dem Plasma chronischer HBsAg-Träger gewonnen wurde. Inzwischen wird der Impfstoff gentechnologisch gewonnen und zur aktiven Immunisierung gefährdeter Personengruppen angewendet. 95 % der vor Impfung seronegativen Personen zeigen nach einem Jahr eine Immunreaktion. Alle verwendeten Hepatitis B Impfstoffe enthalten in hoher Konzentration das gereinigte, der nicht infektiösen Hülle des Hepatitis B Virus entsprechende HBsAg-Protein und sind frei von Virus-DNA bzw. Formalin-inaktiviert.

Ein Nachteil des Standes der Technik besteht darin, dass mindestens 5 % der immunisierten Personen sog. "non-responder" sind, die keine Immunantwort zeigen. Darüber hinaus ist bis heute kein Vakzin zur Behandlung von chronisch persistierender Hepatitis bekannt.

WO 01/40279 und WO 01/38498 beschreiben Vakzine auf der Basis von Hepatitis B Virus Genotyp G. Den beiden Patentschriften ist jedoch kein Hinweis auf die Kombination verschiedener Genotypen zu entnehmen.

Michel et al., PNAS 92 (1995), 5307-5311 und Mancini et al., PNAS 93 (1996), 12496-12501 betreffen DNA-Vakzine auf der Basis von HBsAg. Den Dokumenten ist kein Hinweis auf die Verwendung von Zusammensetzungen zu entnehmen, die Kombinationen von HBsAg unterschiedlicher HBV-Genotypen enthalten.

Der vorliegenden Erfindung liegt daher die Aufgabe zugrunde, verbesserte Mittel zur Prophylaxe/Therapie einer HBV-Infektion oder einer durch HBV-vermittelten Erkrankung bereitzustellen. Der vorliegenden Erfindung liegt ferner die Aufgabe zugrunde, ein Patienten-spezifisches Arzneimittel zur therapeutischen Behandlung von Hepatitis anzugeben.

Die der vorliegenden Erfindung zugrundeliegende Aufgabe wird gelöst durch die Bereitstellung einer Zusammensetzung, wie in Anspruch 1 definiert.

Der vorliegenden Erfindung liegt folgende überraschende Beobachtung zugrunde. Als präklinisches Modell zur Beurteilung der Effizienz spezifischer Immuntherapieprotokolle für chronische HBV-Infektionen sind transgene Mäuse anzusehen, die konstitutiv den HBsAg-Subtyp ayw in der Leber exprimieren. Diese Mäuse produzieren große Mengen an HBsAg, welches aufgrund einer persistierenden Antigenämie auftritt, und sind im wesentlichen tolerant gegenüber HBsAg. Die Erfinder haben nun HBsAg transgene Mäuse einerseits mit einer Vakzine immunisiert, die in ihrem HBsAg Genotyp genau dem Genotyp der transgenen Maus (ayw) entspricht, und andererseits mit einer Vakzine, die einen HBsAg Genotyp enthält, der sich von dem der transgenen Maus unterscheidet. Trotz wiederholter Immunisierung der transgenen Maus mit einem HBsAg-Antigen, der dem eigenen HBsAg entspricht, konnte keine zytotoxische T-Zellantwort beobachtet werden. Im Gegensatz hierzu ergab die Immunisierung der transgenen Mäuse mit einem vom eigenen Genotyp unterschiedlichen HBsAg-Genotyp, Genotyp-spezifische und kreuzreaktive zytotoxische T-Zellantworten gegenüber HBsAg. Dies zeigt, dass eine natürlich vorkommende Variante von HBsAg die "Toleranz" durch das Priming einer kreuzreaktiven T-Zellimmunität durchbrechen kann. Die Aktivierung der zytotoxischen T-Zell-Immunität führt hierbei zu einer Abnahme des HBsAg ayw-Antigens und ferner zu einer Leber-spezifischen Symptomatik, die einer akuten Hepatitis mit einer effektiven Bekämpfung des HBV entspricht. Die beobachtete Immunantwort ist insbesondere bemerkenswert, da sich die Aminosäuresequenz des HBsAg ayw-Antigens nur an wenigen Positionen von der Aminosequenz des HBsAg adw2-Antigens unterscheidet. In der vorliegenden Erfindung wird festgestellt, dass bereits wenige konservative Austausche von Aminosäuren in einem T-Zellepitop zu einer veränderten T-Zellreaktion gegenüber diesem Epitop führen können.

Die Spezifität und Effizienz der T-Zellantwort gegen ein Proteinantigen wird auf unterschiedlichen Ebenen reguliert, insbesondere entscheidend ist (i) die proteolytische Freisetzung des Epitops (bzw. antigenen Peptids); (ii) die Affinität des antigenen Peptids für das präsentierende Glykoprotein des Haupthistokompatibilitätskomplexes (MHC); und (iii) die negative Interferenz kompetitiv sich entwickelnder T-Zellantworten gegen unterschiedliche Epitope desselben Antigens. Natürliche Varianten eines Proteinantigens können (durch individuelle Aminosäureaustausche in kritischen Sequenzen innerhalb des Epitops oder das Epitop flankierend, oder durch Schaffung neuer Epitope) auf folgenden 4 Wegen eine spezifische T-Zellantwort induzieren:
(i) effizientere proteolytische Prozessierung (Freisetzung) des antigenen Peptids aus dem Protein;
(ii) hoch-affine Bindung des antigenen Peptides an das präsentierende MHC Molekül;
(iii) Elimination immundominanter Epitope (die Antworten gegen andere Epitope desselben Proteinantigens supprimieren) durch einen, in (i) und/oder (ii) aufgeführten, analogen Prozess, der die Immunogenität des Epitops abschwächt;
(iv) neue Epitope können generiert werden.

Im Rahmen der vorliegenden Erfindung wird demonstriert, dass natürliche Varianten des HBsAg, reflektiert durch die Genotypen, ein breiteres Spektrum von Spezifitäten in der T-Zellantwort, die sie stimulieren, aufweisen.

Im Zusammenhang mit der vorliegenden Erfindung bedeutet der Ausdruck "HBV Genotyp" die Gesamtheit des Hepatitis B Virus Genoms. Vorzugsweise wird der HBV Genotyp durch Totalsequenzierung und phylogenetische Analyse bestimmt. Zur Zeit sind 8 Standardgenotypen bekannt. Diese 8 Genotypen basieren auf einer Nukleotidvariation von 8% zueinander; siehe Bartholomeusz, Rev. Med. Virol. 13 (2003), 1-14. Vorzugsweise weist der HBV Genotyp A die Referenz-Nukleinsäuresequenz Genbank X02763 bzw. für den HBV Genotyp A_{afr} die Referenz-Nukleinsäuresequenz gemäß Genbank AF297621 auf. Für den HBV Genotyp Bₐ ist die Referenz-Nukleinsäuresequenz Genbank D00330 und für den Genotyp Bj die Referenz-Nukleinsäuresequenz AB073858. Für den HBV Genotyp C ist die Referenz-Nukleinsäuresequenz Genbank AY206389, in bezug auf den Genotyp Cₐᵤₛ die Referenz-Nukleinsäuresequenz gemäß Genbank AB048704. Für den Genotyp D ist die Referenz-Nukleinsäuresequenz Genbank X02496. Die ReferenzNukleinsäuresequenz für den Genotyp E ist X75657. Die Referenznukleinsäuresequenz für den Genotyp F ist X69798. Die Referenz-Nukleinsäuresequenz für den Genotyp G ist AF160501 und die Referenz-Nukleinsäuresequenz für den Genotyp H ist AY090454.

In bezug auf die vorgenannten Genotypen besteht eine gewisse Korrelation zwischen Genotyp und Subtyp wie folgt: Genotyp A korreliert mit Subtyp adw2, ayw1; Genotyp B korreliert mit adw2, ayw1, Genotyp C korreliert mit adw2, adrq+, adrq-, ayr, adr. Genotyp D korreliert mit ayw2, ayw3, ayw4. Genotyp E korreliert mit ayw4. Genotyp F korreliert mit adw4q-, adw2 und ayw4, Genotyp G korreliert mit adw2 und Genotyp H korreliert mit adw4.

Die Bestimmung des HBV-Subtyps eines infizierten Patienten kann serologisch mit Hilfe von mono-spezifischen Antikörpern z. B. anti-d, anti-y, anti-r, anti-a(w) durchgeführt werden. Die Bestimmung kann als Agargeldiffusionstest oder als Radioimmunassay erfolgen; ("HBs Antigen Subtypes", Herausgeber: Courouce, A. M., Holland, P.V., Muller, J.Y. and Soulier, J. P., Bibliotheca Haematologica no. 42, S. Karger, Basel, 1976).

Der Subtyp kann auch durch Sequenzierung der HBsAg-kodierenden DNA aus Patientenserum bestimmt werden. Aus der bestimmten Nukleinsäuresequenz wird dann die Aminosäuresequenz des HBsAg abgeleitet. Die Zuordnung des Subtyps erfolgt dann über die Aminosäuren an den Positionen 122 und 160 wie beschrieben in Magnius, L.O. und Norder, H., "Subtypes, Genotypes and moleculasr epidemiology of the hepatitis B virus as reflected by sequence variability of the S-gene" Intervirology 38(1-2): 24-34.

Im Zusammenhang mit der vorliegenden Erfindung bedeutet der Ausdruck "Hepatitis B Virus Oberflächen-Antigen" (HBsAg) das kleine HBV Oberflächen-Antigen oder S Protein (p24/gp27). HBsAg kann hierbei ferner die Prä-S1- Proteindomäne umfassen. Vorzugsweise besteht HBsAg aus dem S-Protein und/oder der Prä-S1-Proteindomäne.

In bezug auf die Nummerierung von HBsAg wird das System gemäß Kidd-Ljunggren et al., J. Gen. Virol. 83 (2002), 1267-1280 verwendet.

Der Ausdruck "Fragment" umfasst erfindungsgemäß Fragmente von HBsAg. Vorzugsweise umfasst das Fragment mindestens 5 Aminosäuren und enthält ein T-Zellepitop, vorzugsweise mindestens 10, besonders bevorzugt mindestens 20, insbesondere mindestens 50 Aminosäuren. Gemäß einer bevorzugten Ausführungsform enthält die Zusammensetzung wenigstens zwei HBsAg's oder zwei Fragmente davon. Diese Zusammensetzung ist insbesondere als Vakzine auf Polypeptidbasis verwendbar. Besonders bevorzugt in dem Fall, in dem die Zusammensetzung zwei Fragmente enthält, die sich von HBsAg's mit unterschiedlichem HBV Genotyp ableiten, haben das erste und das zweite Fragment wenigstens 20 Aminosäuren gemeinsam, unterscheiden sich jedoch wenigstens durch eine Aminosäure.

Wie vorstehend ausgeführt, besteht eine Erkenntnis der vorliegenden Erfindung darin, dass bereits sehr geringe Unterschiede in einem Antigen (HBsAg) aufgrund unterschiedlicher Genotypen zu modifizierten T-Zellepitopen führen, die sich lediglich geringfügig voneinander unterscheiden, jedoch zu einer dramatischen Veränderung der TZell-Reaktivität führen. Die beiden sich durch wenigstens eine Aminosäure voneinander unterscheidenden Fragmente können daher leicht durch einfachen Sequenzvergleich der bekannten Genotypen in Bezug auf das HBsAg ermittelt werden. Geeignete Fragmente, die sich durch wenigstens eine Aminosäure voneinander unterscheiden, können in der erfindungsgemäßen Zusammensetzung verwendet werden. Vorzugsweise enthalten die Fragmente wenigstens ein T-Zellepitop, besonders bevorzugt ein humanes T-Zellepitop. Verfahren zur Bestimmung von T-Zellepitopen sind bekannt z. B. Lauer et al., J. Virol. 76 (2002), 6104-6113.

Gemäß einer bevorzugten Ausführungsform enthält die Zusammensetzung wenigstens zwei HBsAg's und/oder wenigstens zwei Fragmente davon.

Ferner bevorzugt sind Zusammensetzungen, die wenigstens ein erstes HBsAg oder ein Fragment davon und eine ein zweites HBsAg oder ein Fragment davon kodierende Nukleinsäure enthalten, wobei sich das erste und zweite HBsAg im HBV Genotyp unterscheiden.

Gemäß einer weiteren bevorzugten Ausführungsform enthält die Zusammensetzung wenigstens zwei Nukleinsäuren, die zwei HBsAg's kodieren, wobei sich-die HBsAg's im HBV Genotyp unterscheiden. Die Nukleinsäuren können hierbei auch solche sein, die ein wie vorstehend definiertes Fragment kodieren. Die Nukleinsäuren können virale DNA oder synthetische DNA sein, wobei unter synthetischen DNA-Sequenzen auch solche verstanden werden, die modifizierte Intemukleosid-Bindungen enthalten. Weiterhin kann es sich bei den Nukleinsäuren um RNA-Moleküle handeln, was für die Expression mittels rekombinanter Vektorsysteme erforderlich sein kann. Ferner werden erfindungsgemäß auch gemischte DNA/RNA-Moleküle als Nukleinsäuren in Betracht gezogen.

Gemäß einer bevorzugten Ausführungsform ist der Genotyp ausgewählt aus den bekannten Genotypen A, B, C, D, E, F, G oder H. Im Hinblick auf die jeweilige ReferenzNukleinsäuresequenz wird auf den vorstehenden Definitionsabschnitt verwiesen. Üblicherweise wird der Genotyp hierbei über eine 8 %ige Nukleotid-Variation relativ zur Referenz-Nukleinsäuresequenz bestimmt, d.h. Nukleinsäuren, die wenigstens 92 % Identität mit der Referenz-Nukleinsäuresequenz aufweisen, werden definitionsgemäß ebenso als Genotyp verstanden. Besonders bevorzugt ist die Identität wenigstens 95 %, insbesondere 98 % relativ zur Referenz-Nukleinsäuresequenz. Die "Identität" relativ zur Referenz-Nukleinsäuresequenz wird hierbei mit Hilfe bekannter Verfahren bestimmt. Generell werden besondere Computerprogramme mit Algorithmen unter Berücksichtigung spezieller Erfordernisse verwendet.

Bevorzugte Verfahren zur Bestimmung der Identität erzeugen zunächst die größte Übereinstimmung zwischen den zu vergleichenden Sequenzen. Computerprogramme zur Bestimmung der Identität umfassen, sind jedoch nicht beschränkt auf das GCG-Programmpaket, einschließlich GAP (Deveroy, J. et al., Nucleic Acid Research 12 (1984), 387; Genetics Computer Group University of Wisconsin, Medicine (WI); und BLASTP, BLASTN und FASTA (Altschul, S., et al. J. Mol. Biol. 215 (1990), 403-410. Das BLASTX Programm kann erhalten werden vom National Center For Biotechnology Information (NCBI) und aus weiteren Quellen (BLAST Handbuch, Altschul S. et al., NCBI NLM NIH Bethesda ND 22894; Altschul S. et *al.;* vorstehend). Der bekannte Smith-Waterman Algorithmus kann ebenso zur Bestimmung der Identität verwendet werden.

Bevorzugte Parameter zum Nukleinsäurevergleich umfassen die nachfolgenden:
Algorithmus Needleman und Wunsch, J. Mol. Biol. 48 (1970), 443-453

Vergleichsmatrix:
Matches = +10
Mismatches = 0
Gap penalty: 50
Gap length penalty: 3

Das GAP-Programm ist ebenso zur Verwendung mit den vorstehenden Parametern geeignet. Die vorstehenden Parameter sind die default-Parameter im Nukleinsäuresequenzvergleich. Weitere Beispiele für Algorithmen, Gap Opening Penalties, Gap Extension Penalties und Vergleichsmatrizen umfassen jene in dem Programmhandbuch Wisconsin Package, Version 9, September 1997. Die Wahl hängt hierbei von dem durchführenden Vergleich ab und ferner, ob der Vergleich zwischen Paaren von Sequenzen durchgeführt wird, wenn GAP oder Best Fit verwendet werden oder zwischen einer Sequenz und einer großen Sequenzdatenbank, wenn FASTA oder BLAST verwendet werden.

Eine Übereinstimmung von 92 % gemäß dem vorstehenden Algorithmus bedeutet im Zusammenhang mit der vorliegenden Erfindung 92 % Identität. Das gleiche gilt für höhere Identitäten.

Bevorzugt ist die erfindungsgemäße Zusammensetzung dadurch gekennzeichnet, dass die Variante eine Polymerase kodiert, deren Aktivität im wesentlichen der Aktivität der von der Referenz-Nukleinsäuresequenz kodierten Polymerase entspricht und/oder die Variante ein HBsAg kodiert, dessen Immunreaktivität im wesentlichen der Immunreaktivität des von der Referenz-Nukleinsäure kodierten HBsAg entspricht.

Die Polymeraseaktivität kann hierbei bestimmt werden gemäß Kim et al, Biochem. Mol. Biol. Int. 1999; 47 (2), 301-308. Die Immunreaktivität von HBsAg kann durch kommerziell erhältliche Antigen-ELISA's bestimmt werden. Eine "im wesentlichen von der Immunreaktivität des von der Referenz-Nukleinsäure kodierten HBsAg" bedeutet, dass ein Antikörper an das Referenz HBsAg mit im wesentlichen derselben Affinität wie an das von der Variante kodierte HBsAg bindet.

Gemäß einer bevorzugten Ausführungsform enthält die Zusammensetzung wenigstens drei, vorzugsweise wenigstens fünf unterschiedliche HBsAg's, Fragmente davon und/oder diese kodierende Nukleinsäuren.

Besonders bevorzugt enthält die Zusammensetzung HBsAg's sämtlicher bekannter HBV Genotypen, Fragmente davon und/oder diese kodierende Nukleinsäuren.

Gemäß einer weiteren bevorzugten Ausführungsform der erfindungsgemäßen Zusammensetzung liegt die Nukleinsäure, die HBsAg oder ein Fragment davon kodiert, in einem Vektor unter der Kontrolle eines zur Expression von HBsAg in einer Säugerzelle, vorzugsweise einer humanen Zelle, geeigneten Promotors vor. Sofern die Zusammensetzung wenigstens zwei HBsAg oder ein Fragment davon kodierende Nukleinsäuren enthält, können diese in demselben Vektor (binärer Vektor) oder getrennt voneinander auf verschiedenen Vektoren vorliegen. Geeignete Vektoren sind hierbei bspw. Plasmide, Adenoviren, Vacciniaviren, Baculoviren, Masemviren und Retroviren. Der Vektor umfasst generell einen Replikationsursprung, der die Replikation des Vektors in der transfizierten Säugerzelle bewirkt.

Geeignete Promotoren können sowohl konstitutive als auch induzierbare Promotoren sein. Bevorzugte Promotoren sind abgeleitet von CMV und SV-40.

Die vorstehend beschriebenen Zusammensetzungen können durch einfaches Mischen der einzelnen Komponenten erhalten werden und sind daher sehr einfach herstellbar. Geeignete Lösungsmittel und Träger hängen hierbei von der Art der Zusammensetzung (Polypeptid und/oder Nukleinsäuren) ab. Grundsätzlich sind wasserhaltige Systeme bevorzugt HBsAg oder Fragmente davon sind synthetisch oder durch rekombinante Herstellung erhältlich. Die hergestellten Polypeptide können durch chromatographische Verfahren aufgereinigt werden.

Gemäß einem weiteren Aspekt der vorliegenden Erfindung werden pharmazeutische Zusammensetzungen bereitgestellt, die eine erfindungsgemäße Zusammensetzung und einen pharmazeutisch verträglichen Träger enthalten. Pharmazeutisch verträgliche Träger sind dem Fachmann bekannt. Beispiele sind: Aluminiumsalze, Calciumphosphat, Lyophilisate des HBsAg mit oder ohne Polysaccharidzusatz, Öl-in-Wasser-Emulsionen, Poly-Lactid-Co-glycolat. Diesen Trägern können, soweit nicht selbst schon adjuvantiv wirkend, weitere Adjuvantien beigefügt sein, wie z. B. Lipid A-Mimetika, Immunstimulatorische Nukleotide oder bakterielle Toxine.

Die erfindungsgemäße pharmazeutische Zusammensetzung ist insbesondere ein Vakzin. Erfindungsgemäß wird angegeben, dass die pharmazeutische Zusammensetzung, insbesondere das Vakzin, zur therapeutischen Behandlung einer HBV-Infektion oder einer durch HBV-vermittelten Erkrankung geeignet ist. Ebenso ist die pharmazeutische Zusammensetzung, insbesondere das Vakzin, zur prophylaktischen Behandlung einer HBV-Infektion oder einer durch HBV-vermittelten Erkrankung geeignet Die HBV-Infektion ist vorzugsweise eine chronisch persistierende Hepatitis B-Infektion. Eine HBV-vermittelte Erkrankung kann eine akute chronische Hepatitis B-Infektion sein. Weitere HBV-vermittelte Erkrankungen sind die Leberzirrhose und das primäre Leberzellkarzinom. Das Vakzin ist zur Verabreichung an klinisch inapparente HBV-Träger geeignet, d. h., solche, die noch nicht im eigentlichen Sinne erkrankt sind, jedoch für die Zukunft ein hohes Risiko aufweisen, eine HBV-vermittelte Erkrankung auszubilden.

Die pharmazeutische Zusammensetzung kann intramuskulär, subkutan, intradermal, intravenös, mucosal oder oral verabreicht werden, wobei dies lediglich als bevorzugt angegeben wird und nicht darauf beschränkt ist.

Die pharmazeutische Zusammensetzung enthält die wenigstens zwei HBsAg's oder Fragmente davon im Dosisbereich von 0,1 bis 1000 µg/ HBsAg oder Fragment davon, vorzugsweise 2,5 bis 40 µg/HBsAg oder Fragment davon.

Sofern die pharmazeutische Zusammensetzung, HBsAg oder Fragmente davon kodierende Nukleinsäuren enthält, so liegen diese im Dosisbereich von 10 bis 1000 µg / HBsAg oder Fragment davon kodierende Nukleinsäure.

Gemäß einem weiteren Aspekt der vorliegenden Erfindung wird ein Verfahren zur Herstellung eines Arzneimittels zur therapeutischen-Behandlung von Hepatitis B angegeben, wie in Anspruch 20 definiert.

Wie vorstehend ausgeführt, besteht eine wesentliche Erkenntnis der vorliegenden Erfindung darin, dass in einem präklinischen Modell der chronisch persistierenden Hepatitis ein Behandlungseffekt dadurch erzielt werden konnte, dass das transgene Tier mit einem HBsAg behandelt wurde, das von einem HBV Genotyp stammt, der sich vom Genotyp des transgenen Tieres unterscheidet.

Der Genotyp kann hierbei durch die folgenden Verfahren bestimmt werden: Sequenzierung des vollständigen HBV-Genoms oder wenigstens des für das HBsAg-kodierenden Abschnitts und phylogenetische Analyse, Restriktionsfragmentlängen-Polymorphismus (RFLP), Multiplex-PCR.

Das Bereitstellen des Arzneimittels wird durch Formulieren wenigstens eines HBsAg, eines Fragments davon oder eine HBsAg oder eines Fragments davon kodierende Nukleinsäure in an sich bekannter Weise durchgeführt.

Gemäß einem weiteren Aspekt schlägt die vorliegende Erfindung die Verwendung von wenigstens einem HBsAg vor, wie in Anspruch 22 definiert.

### Fiaurenbeschreibung

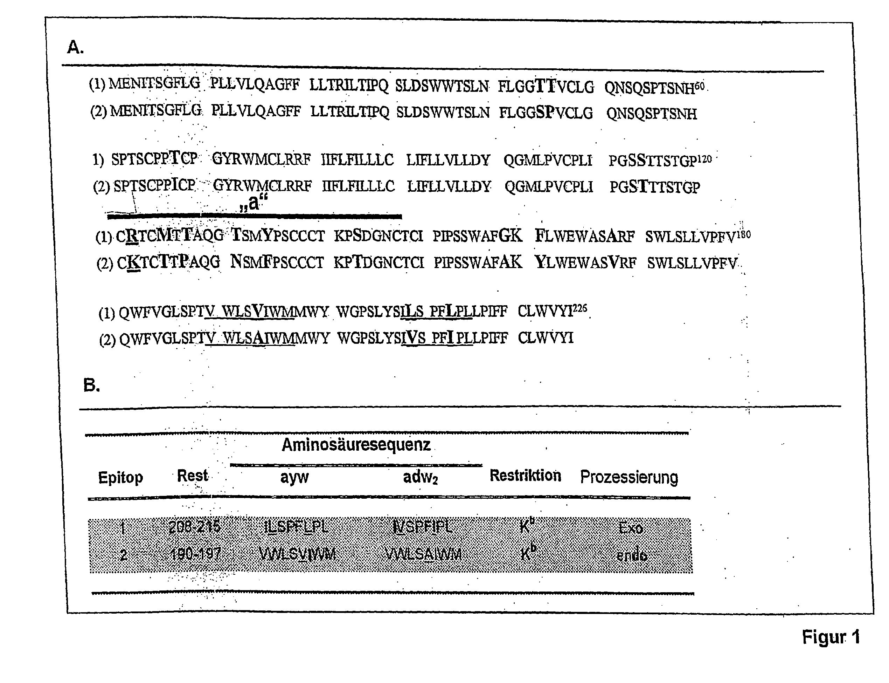

**Figur 1****:** HBsAg Varianten. (A) Die Aminosäuresequenz des kleinen Hepatitis B Oberflächen-Antigens (HBsAg) ayw (1) entsprechende Genotyp **D** und adw2 (2) entsprechend Genotyp **A** sind gezeigt. (B) HBsAg ayw- und adw2-abgeleitete, k^{b}-restringierte Epitopsequenzen. Das Epitop 1 (S₂₀₈₋₂₁₅) wurde nur von den Zellen präsentiert, die exogenes HBsAg prozessieren, wohingegen Epitop 2 (S₁₉₀₋₁₉₇) nur von den Zellen präsentiert wurde, die endogenes HBsAg prozessieren.

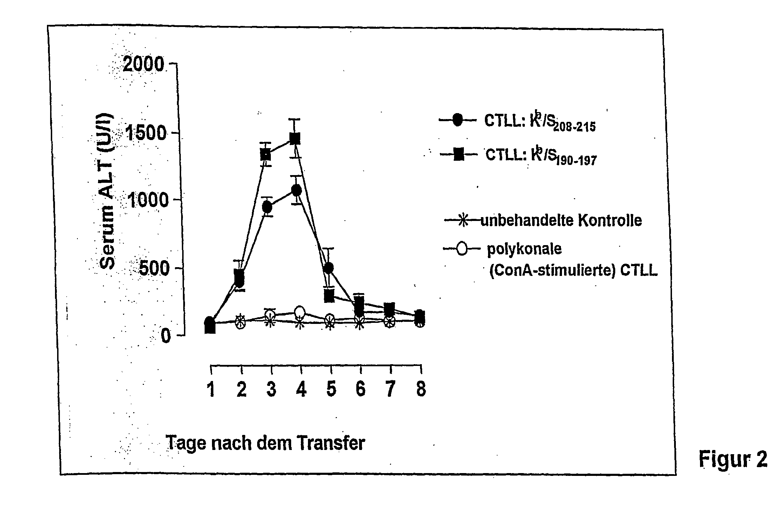

**Figur 2****:** Transfer von Epitop 1- oder Epitop 2-spezifischen zytotoxischen T-Zelllinien (CTLL) in HBs-transgene (HBs-tg) Wirte führen zytotoxische T-Zelllinien HBs-transgene zu transienter Leberschädigung. Die Milzzellen wurden entnommen aus pCI/S_{ayw} DNAimmunisierten B6-Mäusen und *in vitro* mit syngenen, RBL5-Zellen restimuliert, wobei die RBL5-Zellen mit K^{b}/S₂₀₈₋₂₁₅ Bindungspeptid 1 (ILSPFLPL) oder K^{b}/S₁₉₀₋₁₉₇ Bindungspeptid 2 (VWLSVIWM) gepulst waren, oder mit ConA stimuliert wurden. 5 x 10⁶ CD8⁺ CTLL/Maus wurden intravenös (i. v.) in HBs-tg Mäuse injiziert und die durchschnittlichen Serum Alanin-Transaminase (ALT)-Spiegel bestimmt.

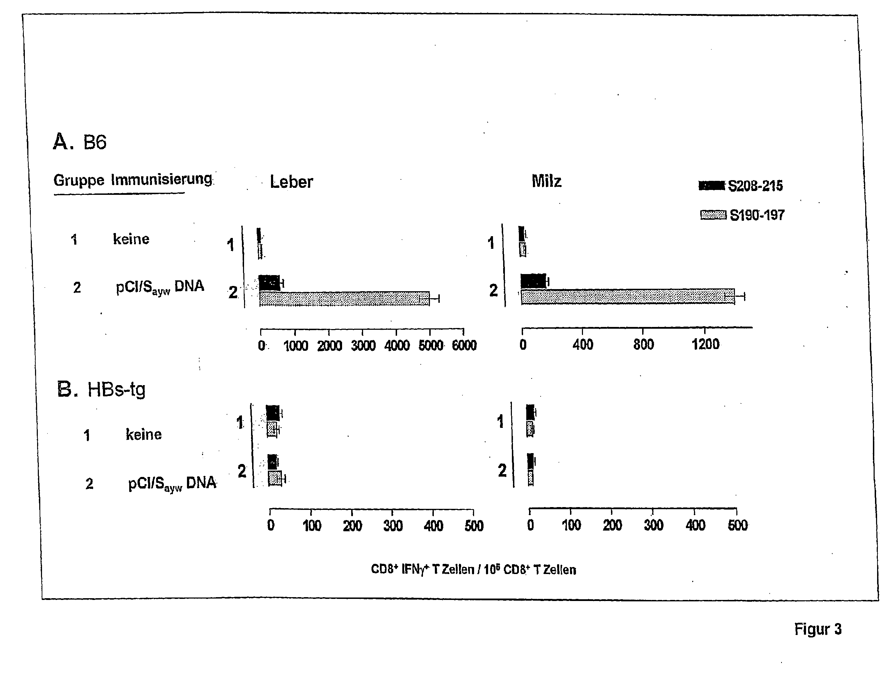

**Figur 3****:** *Ex vivo* Nachweis von HBsAg-spezifischen CD8⁺ T-Zellen in der Leber und Milz immunisierter Mäuse. C57BL/6 Mäuse wurden intramuskulär durch eine einzelne Injektion von 100 µg pCI/S_{ayw}-DNA immunisiert. Spezifische CD8⁺ T-Zellen wurden 12 Tage nach der Immunisierung nachgewiesen. Isolierte Leber-mononukleäre Zellen (MNC) und Milzzellen wurden *in vitro* über vier Stunden (in Gegenwart von Brefeldin A) mit dem K^{b}/S₂₀₈₋₂₁₅ Bindungspeptid 1 (ILSPFLPL) oder dem K^{b}/S₁₉₀₋₁₉₇ Bindungspeptid 2 (VWLSVIWM) restimuliert. Die Durchschnittsfrequenz von CD8⁺ IFNγ⁺ T-Zellen / 10⁵ CD8⁺ T-Zellen ± Standardabweichung von 4-6 Mäusen (aus zwei voneinander unabhängigen Experimenten) pro Gruppe ist gezeigt.

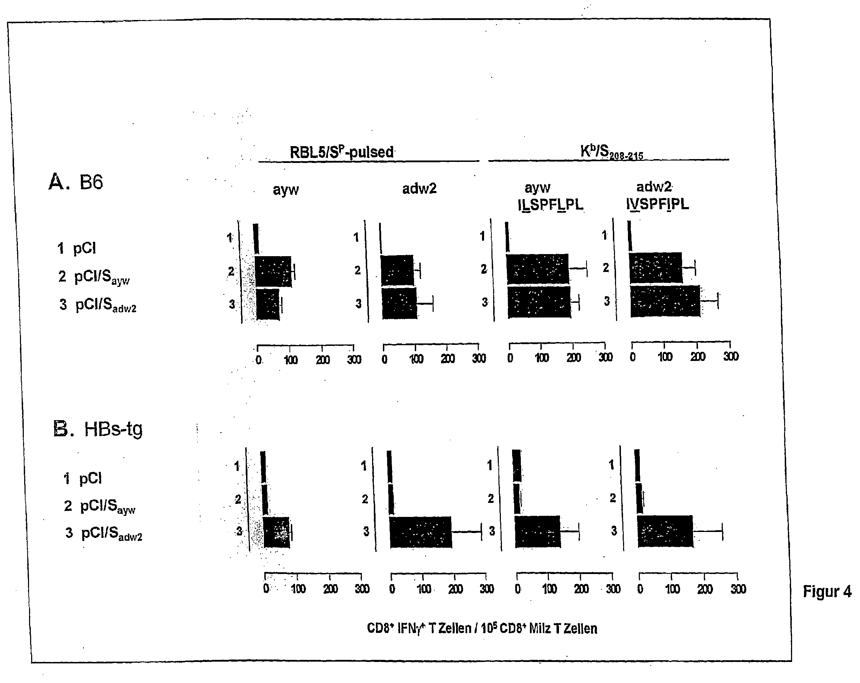

**Figur 4**:HBsAg-spezifische CD⁸ T-Zell-Antworten gegen das Epitop 1 in HBs-tg-Mäusen. HBs-tg-Mäuse, die HBsAg_{ayw} in der Leber exprimieren, wurden intramuskulär dreimal (in vierwöchigem Abstand) mit DNA-Vakzinen immunisiert, die HBsAg Subtyp ayw(pCI/S_{ayw}) oder adw2 (pCI/S_{adw2}) kodieren, oder mit dem Negativ-Kontrollvektor pCI (Vektor ohne Insert). Die Milzzellen wurden aus den immunisierten Mäusen 12 Tage nach der letzten Immunisierung entnommen und wurden über vier Stunden *in vitro* (in Gegenwart von Brefeldin A) mit RBL5-Zellen restimuliert, wobei die RBL5-Zellen mit HBsAg-Partikeln des ayw (RBL5/S^{P}_{ayw}) oder adw2 (RBL5/S^{P}_{adw2}) Subtyp, oder mit dem K^{b}/S₂₀₈₋₂₁₅ Bindungspeptid 1 von HBsAg_{ayw} (ILSPFLPL) oder HBsAg_{adw2} (IVSPFIPL) restimuliert wurden. Die durchschnittliche Anzahl an Milz-IFNγ⁺ CD8⁺ T-Zellen / 10⁵ CD8⁺ T-Zellen ± Standardabweichung von 4-6 Mäusen (aus zwei voneinander unabhängigen Experimenten) pro Gruppe ist gezeigt.

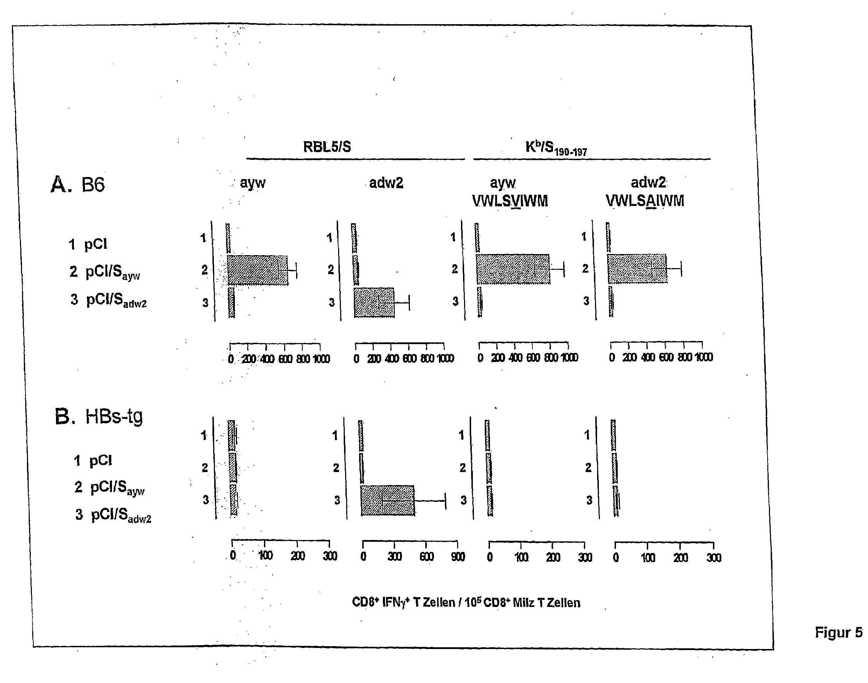

**Figur 5****:** HBsAg-spezifische CD⁸ T-Zell-Antworten gegen Epitop 2 in HBs-tg Mäusen. Die Milzzellen wurden aus Mäusen entnommen, die wie in bezug auf die Legende von Figur 4 beschrieben, immunisiert waren, und *in vitro* mit syngenen RBL5/ S_{ayw} oder RBL5/S_{adw2} -Transfektanten, oder dem K^{b}/ S₁₉₀₋₁₉₇ Epitop 2 von HBsAg_{ayw} (VWLSVIWM) oder HBsAg_{adw2} (VWLSAIWM) restimuliert wurden. Die durchschnittlichen Anzahlen von Milz-IFNγ⁺ CD8⁺ T-Zellen / 10⁵ CD8⁺ T-Zellen ± Standardabweichung von 4 Mäusen pro Gruppe ist gezeigt.

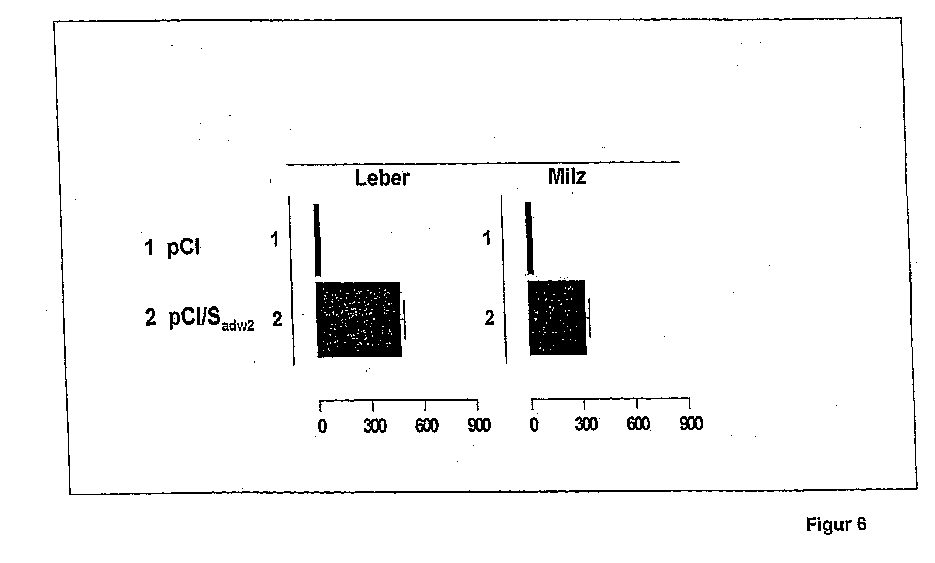

**Figur 6****:** S₂₀₈₋₂₁₅-spezifische CD8⁺ T-Zellen wurden in der Leber von immunisierten HBs-tg-Mäusen nachgewiesen. Transgene HBs-tg-Mäuse wurden dreimal (im Abstand von 4 Wochen) mit einer DNA-Vakzine immunisiert, die HBsAg_{adw2} (pCI/S_{adw2}) kodiert. Leber- und Milzzellen wurden aus immunisierten Mäusen 12 Tage nach der letzten Injektion entnommen und *in vitro* mit dem K^{b}S₂₀₈₋₂₁₅-Bindungspeptid ILSPFLPL restimuliert. Die durchschnittliche Anzahl an Milz-IFNγ⁺ CD8⁺ T-Zellen / 10⁵ CD8⁺ T-Zellen ± Standardabweichung von 4 Mäusen pro Gruppe ist gezeigt.

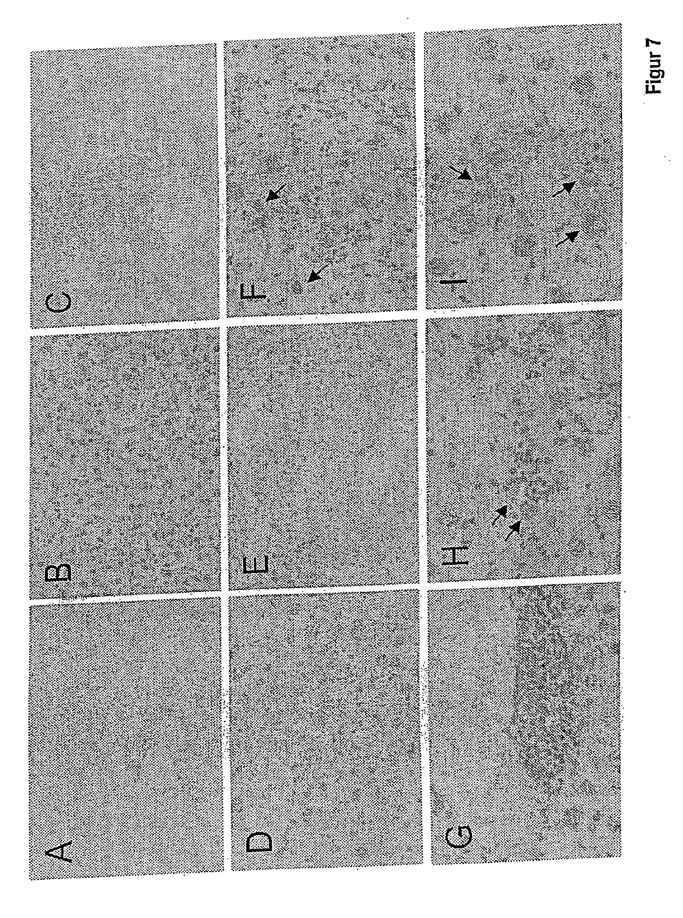

**Figur 7****:** Leberhistopathologie von HBs-tg-Mäusen, die mit dem pCI/Sg_{adw2} DNA -Vakzin immunisiert wurden. Nicht-pathologische Leber-Histologie wurde in B6-Mäusen (A, B) beobachtet. HBs-tg-Mäuse (C, D) zeigten eine moderate Zellvergrößerung und das Cytoplasma zeigt ein milchglasartiges Aussehen (D). Die Kerne der Leberzellen erscheinen moderat polymorph. Periportale Infiltrationen sind selten. Das wiederholte Immunisieren mit pCI/S_{adw2} DNA induziert schwerwiegende histomorphologische Veränderungen der Leber (E-I), die konsistent sind mit akuter viraler Hepatitis. Inflammatorische Infiltrationen umfassen Kupfer-Zellen, Lymphozyten und wenige polymorphnukleäre Granulozyten, die im lobulären Parenchym (F) und in den Periportal- Feldern (G) gelegen sind. Die Hepatozyten erscheinen hydropisch und weisen oft pyknotische Kerne auf, welches ein Anzeichen für ein frühes Stadium der Apoptose darstellt (F, Pfeile). Acidophile Körper (H, Pfeile), d. h., apoptotische Leberzellen sind häufig und oftmals umrundet von fokalen inflammatorischen Infiltrationen. Viele Leberzellkerne zeigen deutliche Vakuolisierung (I, Pfeile). H & E-Färbungen von Formalin-fixiertem, Parafin-eingebettetem Gewebe. Ursprüngliche Vergrößerungen: x 10 in A, C, und E; x 40 in B, D, und F; x 63 in G-I.

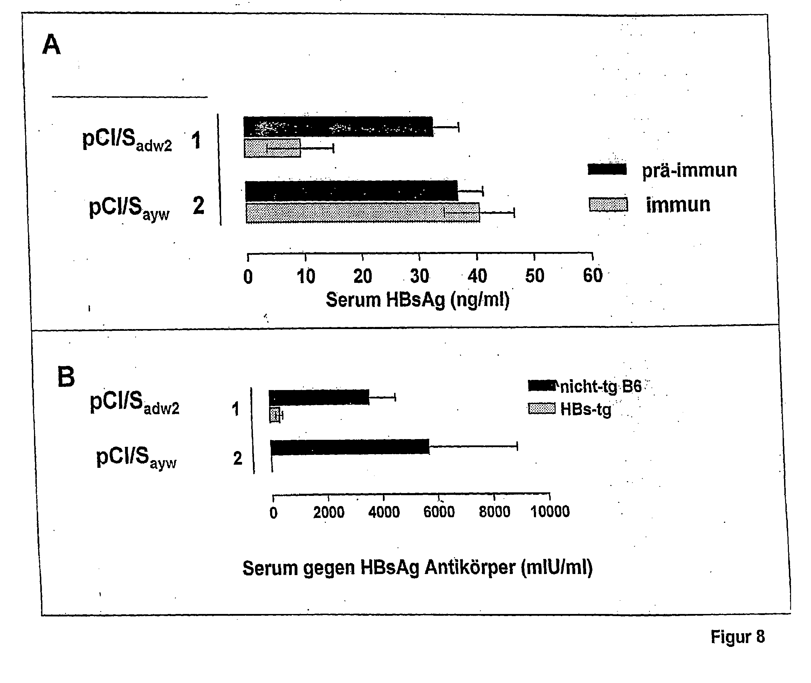

**Figur 8****:** Die Induktion von HBsAg-spezifischen Serumantikörper-Antworten in HBs-tg-Mäusen. B6- und transgene HBs-tg-Mäuse wurden intramuskulär mit DNA-Vakzinen immunisiert, die HBsAg_{adw2} (pCI/S_{adw2}) oder HBsAg_{ayw} (pCI/S_{ayw}) kodieren und nach drei Wochen mit den gleichen Vakzinen geboostet. Vier Wochen nach der letzten Injektion wurden Serumproben auf HBsAg-Antigen (A) oder HBsAg-spezifische Antikörper (B) untersucht. Die durchschnittlichen Antikörper-Titer (mIU/ml) und Serum HBsAg-Spiegel (ng/ml) ± Standardabweichungen von 4-6 Mäusen/Gruppe sind gezeigt.
**Figur 9****:**
   HBsAg-spezifische CD8⁺ T-Zellantworten gegen Epitop 1 (S₂₀₈₋₂₁₅) und gegen Epitop 2 (S₁₉₀₋₁₉₇) in normalen B6 und HBs_{ayw}-tg Mäusen.
   Die Tiere wurden jeweils dreimal (im Abstand von 21 Tagen) intramuskulär mit HBsAg - Proteinpartikeln (S^{P}) des Subtyps ayw bzw. adw2 immunisiert. Den Protein-Vakzinen waren jeweils CpG-Oligonukleotide (ODN) bzw. RC-529 als Adjuvans zugesetzt. Als Negativkontrolle diente PBS. Den Tieren wurde 12 Tage nach der letzten Immunisierung die Milz entnommen und anschließend wurden die vereinzelten Milzzellen über vier Stunden *in vitro* (in Gegenwart von Brefeldin A) mit RBL5 Zellen restimuliert, die zuvor mit HBsAg-spezifischen Peptiden gepulst worden waren. Hierfür wurden jeweils das K^{b}/ S₂₀₈₋₂₁₅ -Bindungspeptid 1 von HBsAg_{ayw} (ILSPFLPL) oder HBsAg_{adw2} (IVSPFIPL) bzw. das K^{b}/ S₁₉₀₋₁₉₇- Bindungspeptid 2 von HBsAg_{ayw} (VWLSVIWM) oder HBsAg_{adw2} (VWLSAIWM) verwendet. Die Anzahl der Milz - IFNγ⁺ CD8⁺ T-Zellen / 10⁵ CD8+ T-Zellen ± Standardweichung von 4 - 6 Mäusen (aus zwei voneinander unabhängigen Experimenten) pro Gruppe ist gezeigt.
**Figur 10****:**
   HBsAg- spezifische CD8⁺ T-Zellantworten gegen das Epitop 1 (S₂₀₈₋₂₁₅) in HBs_{ayw}-tg Mäusen.
      A. HBs-tg-Mäuse, die HBsAg_{ayw} in der Leber exprimieren, wurden intramuskulär dreimal (in vierwöchigem Abstand) mit DNA - Vakzinen immunisiert, die lediglich für HBsAg Subtyp ayw (pCI/S_{ayw}) bzw. die drei Subtypen ayw (pCI/S_{ayw}), adw₂ (pCI/s_{adw2}) und adr (pCI/S_{adr}) kodieren, oder mit dem Negativ-Kontrollvektor pCI (Vektor ohne Insert). Den Tieren wurde 12 Tagen nach der letzten Immunisierung die Milz entnommen. Die vereinzelten Milzzellen wurden über 4 Stunden *in vitro* (in Gegenwart von Brefeldin A) mit RBL5 - Zellen restimuliert, die zuvor mit dem K^{b}/ S₂₀₈₋₂₁₅ -Bindungspeptid 1 von HBsAg_{ayw} (ILSPFLPL) oder HBsAg_{adw2} (IVSPFIPL) gepulst worden waren. Die Anzahl der Milz - IFNγ⁺ CD8⁺ T-Zellen / 10⁵ CD8+ T-Zellen ±Standardweichung von 4-6 Mäusen (aus zwei voneinander unabhängigen Experimenten) pro Gruppe ist gezeigt.
      B. A. HBs_{ayw}-tg-Mäuse wurden intramuskulär dreimal (im Abstand von 21 Tagen) intramuskulär mit HBsAg - Proteinpartikeln (S^{P}) des Subtyps ayw bzw. einer Mischung von HBsAg - Proteinpartikeln der Subtypen ayw,adw2 und adr immunisiert. Den Protein-Vakzinen waren jeweils CpG-Oligonukleotide (ODN) bzw. RC-529 (, nur für Subtyp-Gemisch gezeigt)) als Adjuvans zugesetzt. Als Negativkontrolle diente PBS. Den Tieren wurde 12 Tagen nach der letzten Immunisierung die Milz entnommen. Die vereinzelten Milzzellen wurden über 4 Stunden *in vitro* (in Gegenwart von Brefeldin A) mit RBL5 - Zellen restimuliert, die zuvor mit dem K^{b}/ S₂₀₈₋₂₁₅ -Bindungspeptid 1 von HBsAg_{ayw} (ILSPFLPL) oder HBsAg_{adw2} (IVSPFIPL) gepulst worden waren. Die Anzahl der Milz - IFNγ⁺ CD8⁺ T-Zellen / 10⁵ CD8+ T-Zellen ± Standardweichung von 4-6 Mäusen (aus zwei voneinander unabhängigen Experimenten) pro Gruppe ist gezeigt.
**Figur 11****:**
   Induktion von HBsAg - spezifischen Serumantikörper-Antworten in HBs-tg- Mäusen. B6 und transgene HBs-tg Mäuse wurden intramuskulär mit HBsAg - Proteinpartikel-Vakzinen (S^{P}) des Subtyps ayw oder des Subtyps adw2 oder mit einem Gemisch der Subtypen ayw, adw2 und adr immunisiert und nach drei Wochen mit der gleichen Vakzine geboostet. Die Protein-Vakzine enthielten als Zusatz CpG-Oligonukleotid (ODN) als Adjuvans. Vier Wochen nach der Boost - Injektion wurden Serumproben auf HBsAg (A) und HBsAg- spezifische Antikörper (B) untersucht. Die durchschnittlichen Antikörper-Titer (mIU/ml) und der Serum HBsAg-Spiegel (ng/ml) ± Standardabweichungen von 4-6 Mäusen/ Gruppe sind gezeigt.

Nachstehend wird die Erfindung anhand von Beispielen erläutert. Diese Beispiele sind jedoch nicht beabsichtigt, die Erfindung zu beschränken.

### Beispiele:

### Material und Methoden

### Allgemein

Der untersuchte HBV Subtyp adw2 entspricht dem Genotyp A. Der HBV Subtyp ayw entspricht dem Genotyp D. Der HBV Subtyp adr entspricht dem Genotyp C.

### Mäuse

C57BL/6JBom (B6) Mäuse (H-2^{b}) wurden unter Standardpathogen-freien Bedingungen gehalten.

C57BL/6J-TgN(Alb1 HBV)44Bri Transgene (HBs-tg) Mäuse, die HbsAg_{ayw} (kodiert durch die HBV-Sequenz mit der Hinterlegungsnummer V01460 J02203, wurden vom The Jackson Laboratory (Bar Harbour, ME) bezogen. Männliche und weibliche Mäuse wurden im Alter von 8-16 Wochen verwendet.

### Zellen, rekombinante HBsAg-Partikel und antigene HBsAg-Peptid

Die verwendete H-2^{b}-Zellinie RBL5 ist beschrieben in [10]. Stabile RBL5 Transfektanten, die ähnliche Mengen an HBsAg_{ayw} und HBsAg_{adw2} exprimierten, wurden hergestellt (Daten nicht gezeigt). Rekombinante HBsAg-Partikel der Subtypen ayw, adw₂ und adr sind von der Firma Rhein Biotech GmbH (Düsseldorf, Deutschland) erhältlich. Die in dem *Hansenula polymorpha* Wirtsstamm RB10 hergestellten HBsAg-Partikel wurden wie beschrieben [3], aufgereinigt. Die synthetischen K^{b}-bindenden S₂₀₈₋₂₁₅ ILSPFLPL (ayw) oder I**V**SPF**I**PL (Subtyp adw2) Peptide, und die K^{b}-bindenden S₁₉₀₋₁₉₇ VWLSVIWM (ayw) oder VWLS**A**IWM (adw2) Peptide wurden von Jerini BioTools (Berlin, Deutschland) bezogen. Die Peptide wurden in einer DMSO-Lösung in einer Konzentration von 10 mg/ml gelöst und mit Kulturmedium vor der Verwendung verdünnt.

### Plasmide und DNA-Immunisierung

HBsAg_{ayw} ,HBsAg_{adw2} und HBsAg_{adr} wurden in die pCI (Promega) und BMGneo-Vektoren wie beschrieben [4; 5] kloniert. Als DNA-Vakzine wurden die Plasmide pCI/S_{ayw}, pCI/S_{adw2} pCI/S_{adr} verwendet, die HBsAg_{ayw} ,HBsAg_{adw2} und HBsAg_{adr} gleich gut exprimierten. Dies wurde durch Immunpräzipitation von HBsAg aus Zellen, die transient mit der DNA dieser Plasmide transfiziert waren, gezeigt (Daten nicht gezeigt). Unterschiede in der Immunogenizität der HBsAg-Epitope sind daher nicht aufgrund unterschiedlicher Mengen der HBsAg-Expression durch das DNA-Vakzin oder die Transfektanten zu erklären. Zur intramuskulären Nukleinsäureimmunisierung wurden 50 µl PBS (Phosphat gepufferte Saline) enthaltend 1 µg/µl Plasmid-DNA in jeden Tibialis anterior Muskel wie beschrieben [4] injiziert. Die Immunisierung mit Gemischen von HBsAg Subtypen erfolgte durch Injektion von 50 µl PBS enthaltend jeweils 1µg/µl pCI/S_{ayw}, 1µg/µl pCI/S_{adw2} und 1µg/µl pCI/S_{adr}.

### Immunisierung mit HBsAg - Proteinpartikeln

5 µg HBsAg - Proteinpartikel wurden zusammen mit 30 µg CpG- Oligonukleotid (ODN1826, MWG Biotech, Ebersberg, Deutschland) bzw. 8 µg RC-529( Corixa Corp. Seattle, WA, USA) in 100 µl PBS (Phosphat gepufferte Saline) pro Maus subkutan injiziert. Zur Immunisierung mit einem Gemisch von HBsAg- Subtypen wurden jeweils 5 µg HBsAg_{ayw}, 5 µg HBsAg_{adw2} und 5 µg HBsAg_{adr} - Proteinpartikel zusammen mit 30 µg CpG- Oligonukleotid - Adjuvans bzw. 8µg RC-529 in 100 µl PBS subkutan injiziert.

### Bestimmung der spezifischen Milz- und Leber- CD8⁺-T-Zell-Frequenzen

Milzzellsuspensionen [1] und die Herstellung von hepatischen NPZ (Nichtparenchymalen) Zellen wurde beschrieben [6; 7]. Die Milzzellen und die Leber NPZ (1x10⁶/ml) wurden über 1 Stunde in RPMI-1640 Medium mit 5 µg/µl HBsAg-abgeleiteten Peptiden, oder HBsAg-exprimierenden Transfektanten (10⁶/ml) oder HBsAg Partikel gepulsten Zellen inkubiert. Sodann wurde 5 µg/µl Brefeldin A (BFA) (Katalog Nr. 15870; Sigma) hinzugefügt, und die Kulturen wurden über weitere 4 Stunden inkubiert. Die Zellen wurden geerntet und deren Oberfläche mit anti-CD8 mAb gefärbt, fixiert, permeabilisiert und eine Färbung auf cytoplasmatisches IFNγ durchgeführt. Die Frequenzen von CD8⁺ IFNγ⁺ CTL wurden durch FACS Analyse bestimmt. Der Durchschnittswert für CD8⁺ IFNγ⁺ T-Zellen / 10⁵ Milz- oder Leber-T-Zellen ist gezeigt.

### Transfer von spezifischen CD8⁺-T-Zelllinien

CD8⁺T -Zelllinien wurden aus der Milz von B6-Mäusen erhalten, die mit dem pCI/S_{ayw} DNA-Vakzin immunisiert wurden. Die Milzzellen wurden *in vitro* mit syngenen RBL5-Zellen restimuliert, die mit dem K^{b}/S₂₀₈₋₂₁₅-Bindungspeptid 1 (ILSPFLPL) oder dem K^{b}/S₁₉₀₋₁₉₇-Bindungspeptid 2 (VWLSVIWM) gepulst wurden. In Linien, die über etwa 2 Wochen *in vitro* expandiert wurden, waren mehr als 80 % der CD8⁺T-Zellen von der erwarteten Epitop-Spezifität, wie sich aus den spezifischen IFNγ-Expressionsuntersuchungen ergibt. Die Zellen wurden gewaschen, und 5 x 10⁶ Zellen dieser Linien wurden intravenös injiziert. Kontrollzellen waren nicht-spezifische CD8⁺T-Blasten, die aus 3 Tage ConA-stimulierten Kulturen isoliert wurden.

### Bestimmung von Transaminasen, HBsAg und anti-HBsAg Antikörper im Serum

Serumantikörper wurden wiederholt von einzelnen, immunisierten oder Kontrollmäusen durch Blutentnahme aus der Schwanzvene zu bestimmten Zeitpunkten nach der Injektion erhalten. Die Serum-Alanin-Aminotransferase (ALT)-Aktivität wurde im Blut unter Verwendung des Reflotron^{®} Tests (Katalog Nr. 745138; Roche Diagnostics GmbH) durchgeführt. Die HBsAg-Konzentration im Serum der transgenen Mäuse wurde bestimmt durch den kommerziellen ELISA AUSZYME II (ABBOTT Laboratories, Wiesbaden, Deutschland) Test. Antikörper gegen HBsAg wurden in Mäuseseren unter Verwendung des kommerziellen IMxAUSAB Tests (Katalog Nr. 7A39-20; Abbott, Wiesbaden, Deutschland) nachgewiesen.
Antikörperspiegel wurden mit Hilfe von 6 Standardseren qualifiziert. Die getesteten Seren wurden so verdünnt, dass die gemessenen OD-Werte zwischen dem Standardserum eins und sechs lagen. Die hier gezeigten Werte wurden bestimmt durch Multiplikation der Serumverdünnung mit dem gemessenen Antikörperspiegel (mlU/ml). Die dargestellten Serumtiter entsprechen dem Mittel von 4 einzelnen Mäusen ± Standardabweichung.

### Histologie

Dünne Lebergewebeschnitte (<3mm) wurden in 4 % Formalin (pH 7,0) über 24 Stunden fixiert und in Parafin eingebettet. 2 µm dicke Parafin-Schnitte wurden mit Hämatoxylin-Eosin (H&E) gefärbt.

### Bindung von HBsAg-Peptiden an K^{b}

Affinitätsaufgereinigte MHC Klasse I-Moleküle K^{b} wurden über 48 Stunden bei 18°C mit ansteigenden Konzentrationen von Testpeptid und einer definierten Konzentration (ungefähr 2 nM) radioaktiv markierten VSV NP 52-59 Indikatorpeptid in Gegenwart von 3 µM humanem β2m wie beschrieben [8, 9] inkubiert. Nachfolgend wurde die Bindung der Peptide an MHC Klasse I Moleküle durch Sephadex G50 Säulen-Gelfiltration bestimmt [8]. Das radioaktiv markierte VSV NP 52-59 Peptid befand sich im Ausschlussvolumen (MHC-gebundenes Peptid) und Einschlussvolumen (freies Peptid). Dies wurde bestimmt durch Gamma-Radio-Spektrometrie und der Anteil des Test-Peptides, der an das MHC-Molekül relativ zur Gesamtmenge an Test-Peptid gebunden hatte, wurde bestimmt. Die Konzentration des Test-Peptids, die benötigt wurde, um eine 50%ige Hemmung der Bindung des Indikator-Peptides (IC50-Wert) zu erhalten, wurde bestimmt. Je niedriger der IC50-Wert, umso besser ist die Bindung des Test-Peptides. Um die Depletion an Ligand zu verhindern, wurde in sämtlichen Bindungsexperimenten eine MHC-Konzentration verwendet, die ausreichend war, um nicht mehr als 15-25 % Bindung zu erhalten. Unter diesen Bedingungen ist der IC50-Wert eine Näherung zur Dissoziationskonstante (K_{d}). Sämtliche Bindungsexperimente wurden als Inhibitionsexperimente durchgeführt.

### Beispiel 1

### Adoptiver Transfer von K^{b} restringierten CD8⁺-T-Zelllinien, die spezifisch für das Epitop 1, oder das Epitop 2 sind, induzieren Leberschädigung in HBs-tg B6 Mäusen

Es wurden kurzzeitige CD8⁺-T-Zelllinien erzeugt, die spezifisch für das Epitop 1 oder Epitop 2 (Figur 1B) von HBsAg aus der Milz von B6 Mäusen sind und die immunisiert waren mit pCl/S ayw Plasmid -DNA. Innerhalb dieser Linien waren >95% der Zellen CD8⁺, und die spezifische IFNγ-Expression konnte in >80% dieser CD8⁺-T-Zellen induziert werden. Der adoptive Transfer von 5 x 10⁶ Zellen dieser Linien in kongene B6-Wirte, die HBsAg_{ayw} in der Leber von einem Transgen exprimierten, induzierte akute Leberschädigung, wie sich durch einen kurzen, jedoch hohen Anstieg an Serum-Transaminase ergab (Figur 2). Die Serum-Transaminasespiegel normalisierten sich 5-6 Tage nach dem Transfer und keine transferierten CD8⁺-T-Zellen waren in dem Wirt zu diesem späten Zeitpunkt nachweisbar. Transfer einer gleichen Anzahl an polyklonalen (mitogenaktivierten) CD8⁺-T-Blasten zeigte keine Leberschädigung. Daher wurde festgestellt, dass (i) spezifische CD8⁺-T-Zellen wirksam die Leberschädigung in HBs-tg Mäusen induzieren (wie beschrieben in [2]); (ii) die HBsAg-Epitope, die durch Prozessierung von endogenem oder exogenem HBsAg erzeugt wurden, werden in der Transgenexprimierenden Leber präsentiert; und (iii) adoptiv transferierte CD8⁺-T-Zellen werden rasch aus dem transgenen Wirt entfernt. Transferierte CD8⁺-T-Zellen mit unterschiedlichen Spezifitäten von HBsAg haben daher Zutritt zur Leber und können *in* situ aktiviert werden, können jedoch nicht stabil aufgenommen werden.

### Beispiel 2: K^{b}-restringierte CTL, die die HBsAg Epitope 1 und 2 erkennen, wurden in der Milz und Leber beobachtet

Es wurde untersucht, ob Vakzin-geprimte HBsAg-spezifische CD8⁺-T-Zellen Zugang zur Leber in normalen oder transgenen HBsAg-exprimierenden (HBs-tg) B6-Mäusen (Fig. 3) haben. Milzzellen und nicht-parenchymale Leberzellen (NPZ) wurden aus B6-Mäusen isoliert, die 12-15 Tage zuvor mit dem pCl/S _{ayw} Vakzin immunisiert waren. CD8⁺-T-Zellen, die spezifisch für Epitop 1 oder Epitop 2 waren, wurden in Milz- und Leber- CD8⁺-T-Zellpopulationen aus normal B6 Mäusen gefunden (Fig. 3A). Obwohl die Frequenz an HBsAg-spezifischen CD8⁺-T-Zellen innerhalb der Leber CD8⁺-T-Zellpopulationen hoch war, waren ihre absoluten Zahlen geringer als in der Milz (Daten nicht gezeigt). Im Gegensatz hierzu war keine CD8⁺-T-Zellreaktivität in HBsAg_{ayw} tg B6-Mäusen nachweisbar, die mit dem DNA-Vakzin immunisiert waren, das HBsAg _{ayw} kodiert (Fig. 3B). Weder 3 Boost-Injektionen (in dreiwöchigem Abstand) mit dem DNA-Vakzin, noch wiederholte Immunisierungen mit HBsAg Antigen-Partikeln und Oligonukleotid-Adjuvanz rief HBsAg-spezifische CD8⁺-T-Zellimmunität in HBs-tg-Mäusen hervor (Daten nicht gezeigt). Somit können Impfprotokolle unter Verwendung der gleichen HBsAg-Variante, gegen die die Maus tolerant ist, keine wirksame anti-virale CD8⁺-T-Zell-Immunität primen.

### Beispiel 3: K^{b}-restringierte T-Zellantworten gegen die Epitope der HBSAg_{ayw} und HBsAG_{adw2} -Varianten

Die 226 Aminosäurereste aufweisenden HBsAg_{ayw} und HBsAg _{adw2} -Proteine aus den HBV Isolaten unterscheiden sich in 16 Aminosäureresten (somit eine 93%ige Identität ihrer Aminosäuren). Die Sequenz des HBsAg_{ayw} -Proteins, das verwendet wurde, ist identisch mit der Sequenz des Transgen-kodierten HBsAg_{ayw}, das von den HBs-tg B6-Mäusen exprimiert wurde. Die Sequenzen der K^{b}-Bindungsepitope 1 und 2 von HBsAg_{ayw} und HBsAg_{adw2}, die ausgewählt wurden, unterscheiden sich um 1 bzw. 2 Aminosäurereste innerhalb des Epitops, weisen jedoch identische flankierende Sequenzen auf (Fig. 1A, B). Das S₂₀₈-₂₁₅-Epitop 1 von HBsAg_{ayw} und HBsAg_{adw2} unterscheiden sich in zwei Positionen: in adw2, ist ein Valin (V)-Rest durch ein Leucin (L) an Position 2 substituiert, und ein Isoleucin (I) ist durch einen Leucin (L)-Rest an Position 6 ersetzt (Fig. 1 B). Die Bindungsaffinität des Epitops 1 von K^{b} war ziemlich gering; die HBsAg_{adw2}-Variante von Epitop 1 zeigte eine höhere Bindungsaffinität an K^{b} als die HBsAg _{ayw}-Variante des Epitops (Tabelle 1). Im Gegensatz hierzu war die Bindungsaffinität von Epitop 2 an K^{b} hoch (Tabelle 1).

**Tabelle 1: Bindungsaffinität von immunogenen HBsAg Epitopen an K^{b}**

| Epitop | HBsAg Variante | Peptid Sequenz | K^{b}-Bindung (nM) |
|---|---|---|---|
| 1 | ayw | I**L**SPF**L**PL | 3400 |
| 1 | adw2 | I**V**SPF**I**PL | 773 |
| **2** | ayw | VWLSVIWM | 54 |

B6-Mäuse, die mit dem pCI/S_{ayw} oder pCl/S_{adw2} DNA -Vakzin immunisiert wurden, zeigten eine CD8⁺-T-Zellantwort gegenüber dem K^{b}-Bindungsepitop 1, die nach einer 5 Stunden ex *vivo* Restimulierung von geprimten Milz CD8⁺-T-Zellen beobachtet wurde, die mit entweder HBsAg_{ayw} oder HBsAg _{adw2}-Partikeln oder Antigen-Peptid S₂₀₈₋₂₁₅ von HBsAg_{ayw} oder HBsAg_{adw2} gepulst waren (Fig. 4A), Gruppe 2,3). Die ayw- und adw2-Variante des Epitops 1 waren kreuzreaktiv, da (i) Epitop 1-spezifische CTL durch pCI/S_{ayw}, oder pCI/S_{adw2} geprimt wurden; und (ii) Zellen, die mit HBsAg_{ayw}, oder HBsA-g_{adw2}-Partikeln gepulst waren oder mit Peptid ILSPFLPL (ayw) oder Peptid IVSPFIPL (adw2) gepulst waren, präsentierten das Epitop 1 gegenüber geprimten CD8⁺-T-Zellen. Somit hemmten die beiden Substitutionen innerhalb des 8mer-Epitops 1 nicht die wirksame Prozessierung, K^{b}-Bindung oder Präsentation des Epitops.

CD8⁺-T-Zellen, die mit dem pCI/S_{ayw} DNA -Vakzin geprimt waren, erkannten das Epitop 2 (S₁₉₀₋₁₉₇) von HBsAg_{ayw}, oder HBsAg_{adw2} (Fig. 5A; Gruppe 2). Dies wurde ex *vivo* nachgewiesen nach einer 5 Stunden-Restimulation unter Verwendung von Peptid-gepulsten Zellen oder Transfektanten, die HBsAg_{ayw} exprimierten. Geprimte CD8⁺-T-Zellen erkannten keine Transfektanten, die das endogene HBsAg_{adw2} exprimierten. Die Immunisierung mit dem pCI/S_{adw2} DNA-Vakzin primte nicht Epitop-2-spezifische T-Zellen (Fig. 5A, Gruppe 3). CD8⁺-T-Zellen, die geprimt waren mit pCI/S_{adw2} (jedoch nicht mit pCI/S_{ayw}) DNA-Vakzin, erkannten ein adw2-spezifisches Epitop unbekannter Epitop/Restriktions-Spezifizität, das von den Transfektanten präsentiert wurde, dies wurde nicht weiter untersucht (Fig. 5, Gruppe 3). Die Substitution der Aminosäure an Position 5 (Austausch der hydrophoben Aminosäure Valin V gegen die hydrophobe Aminosäure Alanin A) hemmt daher die Herstellung des Epitops 2, jedoch nicht dessen Präsentation durch das K^{b}-Molekül ([1].

### Beispiel 4: Kreuz-reaktive K^{b}-restringierte CD8⁺-T-Zellantworten gegen HBsAg-Epitop 1 werden in HBs-tg B6-Mäusen geprimt

HBs-tg B6-Mäuse exprimieren HBsAg_{ayw} von einem Transgen in der Leber. HBs-tg-Mäuse wurden mit HBsAg_{ayw} (pCI/S_{ayw}) oder HBsAg_{adw2} (pCI/S_{adw2}) immunisiert (Fig. 4, 5B). Keine CD8⁺-T-Zellantwort wurde durch wiederholtes Immunisieren von HBs-tg B6-Mäusen mit dem pCI/S_{ayw} DNA -Vakzin erhalten (Fig. 4, 5B, Gruppe2). Im Gegensatz hierzu erzeugte die Immunisierung von HBs-tg B6-Mäusen mit dem pCI/S_{adw2} DNA-Vakzin eine. CD8⁺-T-Zellantwort gegenüber HBsAg (Fig. 4B, Gruppe 3). Diese kreuzreaktive CD8⁺-T-Zellantwort erkannte Zellen, die mit HBsAg _{ayw}- oder HBsAg_{adw2}-Partikeln oder mit der ayw- oder adw2-Variante von Epitop 1 in Peptid-Form gepulst waren (Fig. 4B, Gruppe 3). Diese CD8⁺-T-Zellen erkannten nicht die RBL5/S_{ayw}-Transfektanten oder das K^{b}-Bindungsepitop 2 S₁₉₀₋₁₉₇ (Fig. 5B, Gruppe 3). Die CD8⁺-T-Zellen zeigten eine Subtyp-spezifische Reaktivität gegen eine unbestimmte Determinante, die durch RBL5/S_{adw2} jedoch nicht von den RBLS/S_{ayw} Transfektanten präsentiert wurden (Fig. 5B, Gruppe 3). Dies zeigt, dass eine natürliche Variante von HBsAg die "Toleranz brechen" kann durch das Priming einer kreuz-reaktiven T-Zell-Immunität.

Es wurde untersucht, ob spezifische CD8⁺-T-Zellpopulationen in der Antigenproduzierenden Leber in den transgenen Mäusen nachgewiesen werden können, die mit pCI/S_{adw2} immunisiert wurden. In der Milz und in Leber NPZ aus HBs-tg B6-Mäusen, die mit pCI/S_{adw2} immunisiert waren, konnte spezifische CD8⁺-T-Zellreaktivität über Monate hinweg nachgewiesen werden (Fig. 6). Im Gegensatz zu den adoptiv transferierten CD8⁺-T-Zellen (Fig. 2), haben Vakzin-geprimte anti-HBV-spezifische CD8⁺-T-Zellen daher Zugang und zeigen stabile Aufnahme in das Antigen-tragende Zielorgan über mehr als 3 Monate.

### Beispiel 5: Histopathologie der Leber von immunisierten HBs-tg-Mäusen, die eine spezifische CD8⁺-T-Zellreaktivität gegen das HBsAg-Epitop 1 zeigen

HBsAg-spezifische CD8⁺-T-Zellen induzierten eine inflammatorische Antwort in der HBsAg-produzierenden Leber. Unbehandelte B6-Mäuse zeigten eine normale Leber-Histologie (Fig. 7A, B). Hepatozyten aus HBs-tg B6-Mäusen waren vergrößert und zeigten ein feingranuläres, blass-eosinophiles Zytoplasma, das charakteristisch ist für "Milchglas-Leberzellen", das auch bei der humanen HBV-Infektion beobachtet wird (Figur 7C, D). Keine inflammatorischen Infiltrationen wurden-beobachtet.

HBs-tg-Mäuse, die mit pCI/S_{adw2} (jedoch nicht mit pCI/S_{ayw}) DNA-Vakzin immunisiert waren, zeigten eine schwerwiegende Leber-Histopathologie (Fig. 7E). Inflammatorische Infiltrate, die in den Parenchym- (Fig. 7F) und Periportal (Fig. 7G)-Feldern gefunden wurden, bestanden hauptsächlich aus mononukleären Zellen (Fig. 7F). Kleine, lymphoide Zellen waren zahlreich verteilt in den Parenchym- und Periportal-Feldern. Umschriebene Herde von inflammatorischen Zellen umgaben die apoptotischen Hepatozyten (Fig. 7H). Die Vergrößerung und hydropische Anschwellung von Hepatozyten war stärker in immunisierten als in nicht behandelten HBs-tg Mäusen. Einige mittelgroße bis kleine Kerne zeigten ein kondensiertes Chromatin und einen perinukleären Halo (Fig. 7F Pfeile), das auf ein frühes Stadium der Apoptose hinweist. Ferner wurden zahlreiche Councilman bodies, die apoptotische Leberzellen darstellen, beobachtet (Fig. 7H, Pfeile). Einige Hepatozyten zeigten nukleäre Vakuolisierung (Fig. 7, Pfeile). Eine signifikante Cholestasis war nicht nachweisbar.

### Beispiel 6: Das Priming von HBsAg-spezifischen CD8⁺-T-Zellen in HBs-tg Mäusen korreliert mit einer Reduktion der Antigenämie

Unbehandelte HBs-tg Mäuse zeigen HBsAg Serumspiegel von 30-50 ng/ml (Fig. 8A). Mäuse, die kreuzreaktive CD8⁺-T-Zellantworten gegen Epitop 1 nach der HBsAg_{adw2}-Immunisierung entwickelten, zeigten eine verringerte Antigenämie (mit Spiegeln im Bereich von 5-15 ng/ml), wohingegen Tiere, die mit HBsAg_{ayw} immunisiert waren, die keine HBsAg-spezifische CD8⁺-T-Zell-Immunität entwickelten, keine Veränderung der Antigenämiespiegel (Fig. 8A) zeigten. Die teilweise Kontrolle der Antigenämie korreliert daher mit dem Auftreten von spezifischen CD8⁺-T-Zellen in den immunisierten transgenen Mäusen.

### Beispiel 7: Anti-HBsAg Serum-Antikörper treten in HBs_{ayw}-tg Mäusen auf, die mit HBsAg_{adw2} immunisiert waren

Zusätzlich zur T-Zell-Immunität kann die humorale anti-HBsAg-Immunität eine Rolle bei der Kontrolle der Antigenämie spielen. Es wurde das Auftreten von Anti-HBsAg SerumAntikörpern in vakzinierten normalen und transgenen Mäusen beobachtet. Normale (nicht transgene) B6 Mäuse und kongene HBs-tg B6-Mäuse wurden mit dem pCL/S_{ayw} oder pCL/S_{adw2} DNA-Vakzin zweimal immunisiert. Ihre Serum-Antikörper-Titer, die spezifisch für HBsAg waren, wurden zwei Wochen nach der letzten Immunisierung mit dem ImxAUSAB-Test (Abbott) bestimmt, der HBsAg unterschiedlicher Subtypen bestimmt. Während nicht-transgene Mäuse, die mit pCL/S_{ayw}- oder pCL/S_{adw2}-Plasmid-DNA immunisiert waren, hohe Serum-Antikörperspiegel gegen HBsAg entwickelten, zeigten HBs-tg Mäuse eine anti-HBsAg Serum-Antikörper-Antwort nur nach Immunisierungen mit pCL/S_{adw2} (jedoch nicht mit pCL/S_{ayw}) Plasmid-DNA (Fig. 8B). Ähnliche Antikörper-Antworten wurden in Mäusen beobachtet, die mit HBsAg_{ayw} oder HBsAg_{adw2}-Partikeln immunisiert wurden (Daten nicht gezeigt). Ein Subtyp-spezifischer ELISA (mit HBsAg_{ayw} oder HBsAg_{adw2} Partikel-beschichteten Platten), zeigte, dass in normalen Mäusen >95 % der durch sämtliche Vakzine erzeugten Antikörper-Antwort gegen die 'a'-Determinante von HBsAg gerichtet ist; in HBs-tg-Mäusen ist >90 % der Antikörper-Antwort gegen adw2-spezifische Determinanten (Daten nicht gezeigt) gerichtet.

### Beispiel 8: Effizientes Priming von kreuz-reaktiven K^{b}-restringierten CD8+-T-Zellantworten gegen HBsAg Epitop 1 in HBs-tg B6-Mäusen durch Immunisierung mit HBsAg - Proteinpartikeln

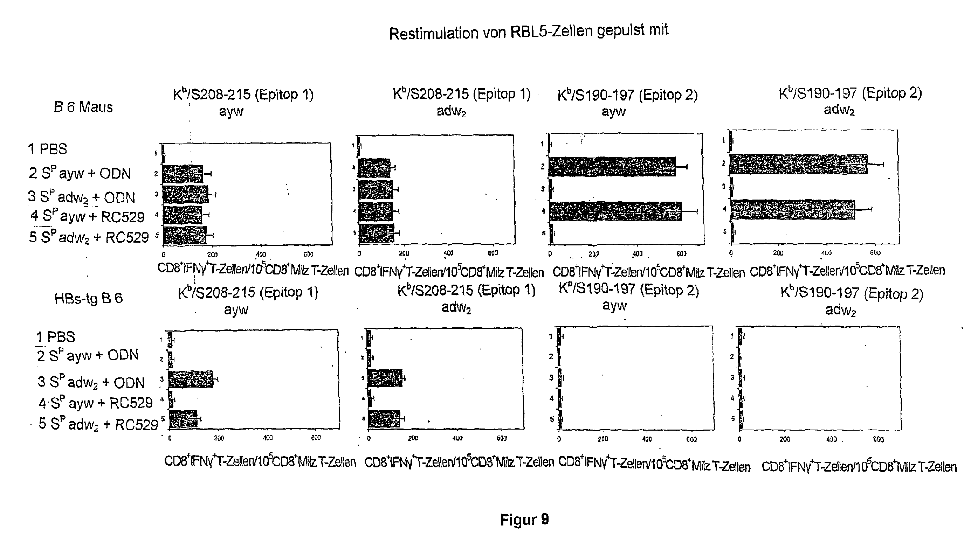

Immunisierung von normalen B6 - Mäusen mit HBsAg-Proteinpartikeln vom Subtyp ayw bzw. adw2 ergeben eine CD8⁺-T-zellvermittelte Immunantwort gegen das K^{b}-Bindungsepitop 1 (S₂₀₈₋₂₁₅). Figur 9A). Damit konnte gezeigt werden, dass unabhängig von der Art der Vakzine (Proteinpartikel oder DNA) Epitope mit unterschiedlichen Sequenzen kreuz-reaktive T-Zellantworten primen können. Analog zu den Immunisierungen mit DNA - Vakzinen (Figur 5) zeigte sich, dass Vakzinierung von B6 Mäusen mit HBsAg-Proteinpartikeln vom Subtyp ayw eine CD8⁺ - Zellantwort gegen das HBsAg- K^{b}-Bindungsepitop 2 (S₁₉₀₋₁₉₇) primt, jedoch nicht Vakzinierung mit HBsAg Proteinpartikeln vom Subtyp adw2 (Figur 9A).
HBs_{ayw}-tg-Mäuse wurden mit HBsAg-Proteinpartikel-Vakzinen, die entweder dem Subtyp ayw oder dem Subtyp adw2 entsprachen, immunisiert. Während nach mehrmaliger Immunisierung mit der HBsAg_{ayw}-Protein-Vakzine keine CD8+-T-Zellantwort generiert wurde, generierte die Immunisierung mit dem heterologen HBsAg_{adw} - Proteinantigen eine HBsAg-spezifische CD8+-T-Zellantwort gegen Epitop 1 (Figur 9B). Damit ist gezeigt, dass eine natürliche Variante von HBsAg auch über eine Protein-Subunit-Vakzinierung durch das Priming einer kreuz-reaktiven T-Zellantwort eine bestehende Toleranz durchbrechen kann.

### Beispiel 9: Effizientes Priming von kreuz-reaktiven K^{b}-restingierten CD8⁺-T-Zellantworten gegen HBsAg Epitop 1 in HBs-tg B6-Mäusen durch Immunisierung mit Gemischen von natürlichen Varianten von HBsAg

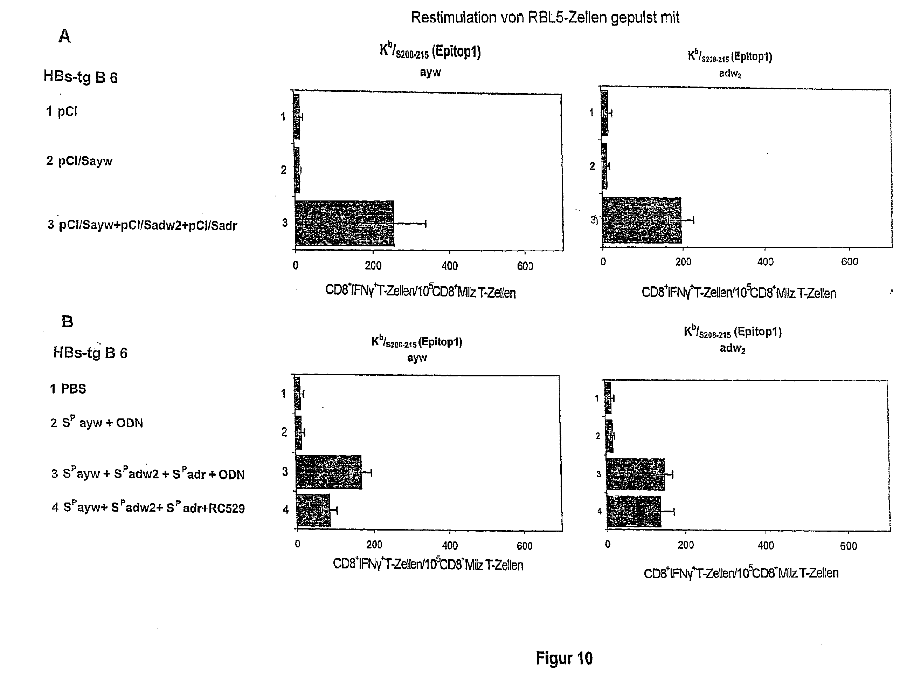

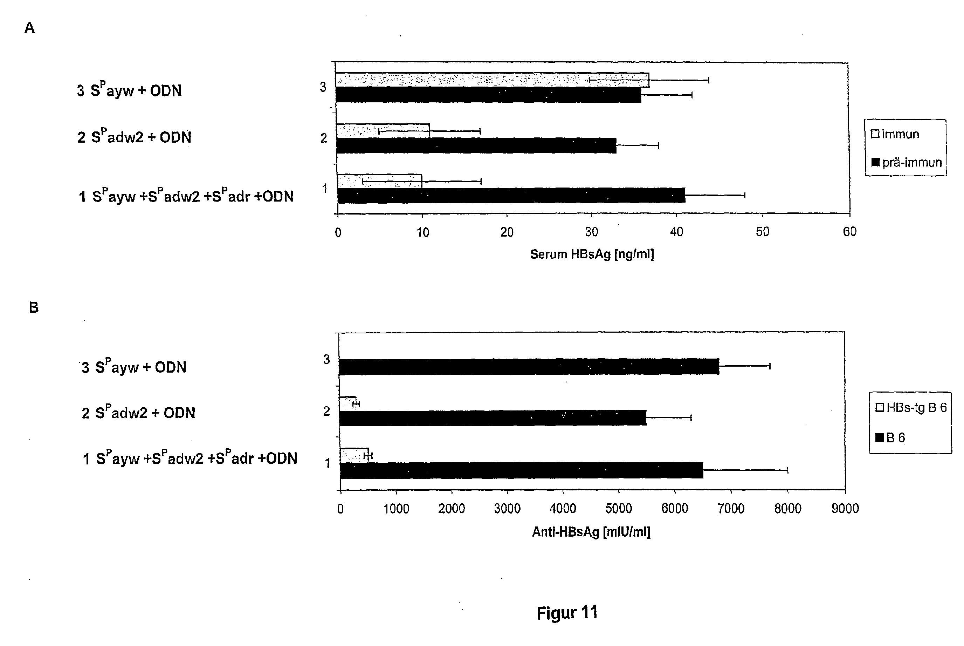

HBs_{ayw}-tg Mäuse wurden nun entweder mit einer DNA - Vakzine, die für die drei HBsAg Subtypen ayw (pCl/S_{ayw}), adw₂ (pCI/S_{adw2}) und adr (pCI/S_{adr}) kodierte (Figur 10A), sowie einer HBsAg- Proteinpartikel-Vakzine, die ein Gemisch der Subtypen ayw, adw₂ und adr enthielt (Figur 10 B), immunisiert. Das Gemisch von natürlichen Varianten von HBsAg primte kreuz-reaktive K^{b}-restringierte CD8+- T- Zellantworten gegen Epitop 1 sowohl nach Immunisierung mit DNA als auch mit Proteinpartikeln.

### Beispiel 10: Reduktion der Anticienämie in HBs-tg-Mäusen nach Immunisierung mit Gemischen von natürlichen Varianten von HBsAg

In unbehandelten HBs-tg-Mäusen wird ein Serumspiegel von 30 - 50 ng/ ml beobachtet. Tiere, die nach Immunisierung mit einer heterologen HBsAg - Vakzine (HBsAg_{adw2}) bzw. einem Gemisch aus natürlichen HBsAg - Varianten (HBsAg_{ayw} + HBsAg_{adw2} + HBsAg_{adr}) eine kreuz-reaktive CD8+ -T-Zellantworten gegen Epitop 1 entwickeln, zeigen eine verringerte Antigenämie (mit HBsAg-Spiegeln von 5- 17 ng/ml). In Tieren, die allein mit dem homologen HBsAg_{ayw} immunisiert wurden und damit keine HBsAg- spezifische T-Zellimmunität generieren konnten, wurde keine Veränderung der Antigenmenge im Serum beobachtet. Die Immunisierung mit einem Gemisch aus natürlichen Varianten von HBsAg kann demnach eine Reduktion der Antigenämie bewirken.

### Beispiel 11: Induktion von anti- HBsAg Serumantikörpern in HBs-tg-Mäusen nach Immunisierung mit Gemischen von natürlichen Varianten von HBsAg

Normale B6 Mäuse zeigen nach Immunisierung mit HBsAg_{ayw}, HBsAg_{adw2}, HBsAg_{adr} (nicht gezeigt) sowie mit einem Gemisch der drei Subtypen eine ausgeprägte Antikörperantwort.
Die Bildung von HBsAg- spezifischen Serumantikörpern in HBs-tg-Mäusen nach Immunisierung wurde untersucht. HBs-tg-Mäuse zeigten nur nach Immunisierung mit einem Gemisch von natürlichen HBsAg - Varianten bzw. mit dem heterologen Subtyp adw₂ eine Serum- Antikörperantwort. Nach Immunisierung mit dem homologen Subtyp ayw wurden keine anti-HBsAg-Antwort induziert. Ein Subtyp spezifischer ELISA (mit HBsAg_{ayw} und HBsAg_{adw2} - Proteinpartikeln beschichteten Mikrotiterplatten) zeigte, dass in HBs-tg - Mäusen >90 % der HBsAg - spezifischen Antikörperantwort gegen adw2 spezifischen Determinanten gerichtet ist (Daten nicht gezeigt).

### Referenzen

1. Schirmbeck,R., Boehm,W., Fissolo,N., Melber,K., and Reimann,J., Different immunogenicity of H-2 Kb-restricted epitopes in natural variants of the hepatitis B surface antigen. Eur.J.Immuno/. 2003. in press: xx-yy.
2. Ando,K.-I., Guidotti,L.G., Wirth,S., Ishikawa,T., Missale,G., Moriyama,T., Schreiber,R.D., Schlicht,H.J., Huang,S.N., and Chisari,F.V., Class I-restricted cytotoxic T lymphocytes are directly cytopathic for their target cells in vivo. J.Immunol. 1994. 152: 3245-3253.
3. S. Schaefer, M. Piontek, S.J. Ahn, A. Papendieck, Z. Janowicz, I. Timmermans, and G. Gellissen. 2002. Recombinant hepatitis B vaccines - disease characterization and vaccine production. in Hansenula polymorpha - Biology and Applications. G. Gellissen (ed.) pp 175 - 210, Wiley-VCH, Weinheim, Germany
4. Schirmbeck,R., Boehm,W., Ando,K.-I., Chisari,F.V., and Reimann,J., Nucleic acid vaccination primes hepatitis B surface antigen-specific cytotoxic T lymphocytes in nonresponder mice. J.Virol. 1995. 69: 5929-5934.
5. Boehm,W., Kuhröber,A., Paier,T., Mertens,T., Reimann,J., and Schirmbeck,R., DNA vector constructs that prime hepatitis B surface antigen-specific cytotoxic T lymphocyte and antibody responses in mice after intramuscular injection. J.Immunol.Methods 1996. 193: 29-40.
6. Trobonjaca,Z., Leithäuser,F., Moller,P., Schirmbeck,R., and Reimann,J., Activating immunity in the liver. I. Liver dendritic cells (but not hepatocytes) are potent activators of IFNγ release by liver NKT cells. J.Immunol. 2001. 167: 1413-1422.
7. Trobonjaca,Z., Kroger,A., Stober,D., Leithäuser,F., Moller,P., Hauser,H., Schirmbeck,R., and Reimann,J., Activating immunity in the liver. II. IFN-β attenuates NK cell-dependent liver injury triggered by liver NKT cell activation. J.Immunol. 2002. 168: 3763-3770.
8. Buus,S., Stryhn,A., Winther,K., Kirkby,N., and Pedersen,L.O., Receptor-ligand interactions measured by an improved spun column chromatography technique. A high efficiency and high throughput size separation method. Biochim.Biophys.Acta 1995. 1243: 453-460.
9. Olsen,A.C., Pedersen,L.O., Hansen,A.S., Nissen,M.H., Olsen,M., Hansen,P.R., Holm,A., and Buus,S., A quantitative assay to measure the interaction between immunogenic peptides and purified class I major histocompatibility complex molecules. Eur.J.Immunol. 1994. 24: 385-392.
10. T. van-Hall, J. van-Bergen, P.A. van-Veelen, M. Kraakman, L.C. Heukamp, F. Koning, C. J. Melief, F. Ossendorp, and R. Offringa. 2000. Identification of a novel tumor-specific CTL epitope presented by RMA, EL-4, and MLB-2 lymphomas reveals their common origin. J. Immunol. 165:869-877

## Patentansprüche

1. Verwendung einer Zusammensetzung, die wenigstens zwei Hepatitis B Virus Oberflächen-Antigene (HBsAg's), Fragmente davon, welche ein T-Zellepitop enthalten, wobei die Fragmente der wenigstens zwei HBsAg's mindestens 10 Aminosäuren gemeinsam haben, jedoch sich wenigstens durch eine Aminosäure voneinander unterscheiden, und/oder diese kodierende Nukleinsäuren enthält, wobei die HBsAg's aus dem S Protein (kleines HBV Oberflächenantigen) bestehen und wobei sich die HBsAg's im Hepatitis B Virus (HBV) Genotyp in der S-Region von HBsAg unterscheiden, und wobei die Zusammensetzung kein HBV Core Antigen (HBcAg) oder dieses kodierende Nukleinsäure enthält, zur Herstellung eines Arzneimittels zur therapeutischen Behandlung einer HBV-Infektion oder einer durch HBV vermittelten Erkrankung.

2. Verwendung einer Zusammensetzung gemäß Anspruch 1, **dadurch gekennzeichnet, dass** die Zusammensetzung wenigstens zwei HBsAg's und/oder wenigstens zwei Fragmente davon enthält.

3. Verwendung einer Zusammensetzung gemäß Anspruch 1 oder 2, **dadurch gekennzeichnet, dass** das/die Fragment/Fragmente von HBsAg mindestens 20, insbesondere mindestens 50 Aminosäuren enthäldenthalten.

4. Verwendung einer Zusammensetzung gemäß Anspruch 3, **dadurch gekennzeichnet, dass** das Fragment die "a-Determinante" von HBsAg umfasst.

5. Verwendung einer Zusammensetzung gemäß einem der Ansprüche 1 bis 4, **dadurch gekennzeichnet, dass** das erste und das zweite Fragment mindestens 20 Aminosäuren gemeinsam haben, jedoch sich wenigstens durch eine Aminosäure voneinander unterscheiden.

6. Verwendung einer Zusammensetzung gemäß Anspruch 1, **dadurch gekennzeichnet, dass** die Zusammensetzung wenigstens zwei HBsAg's oder Fragmente davon kodierende Nukleinsäuren enthält.

7. Verwendung einer Zusammensetzung gemäß einem der Ansprüche 1 bis 6, **dadurch gekennzeichnet, dass** der Genotyp ausgewählt ist aus: A, B, C, D, E, F, G oder H.

8. Verwendung einer Zusammensetzung gemäß einem der Ansprüche 1 bis 7, **dadurch gekennzeichnet, dass** die Zusammensetzung wenigstens 3, vorzugsweise 5 unterschiedliche HBsAg's, Fragmente davon und/oder diese kodierende Nukleinsäuren enthält.

9. Verwendung einer Zusammensetzung gemäß einem der Ansprüche 1 bis 8, **dadurch gekennzeichnet, dass** die Zusammensetzung HBsAg's der Genotypen A, B, C, D, E, F, G und H, Fragmente davon und/oder diese kodierende Nukleinsäuren enthält.

10. Verwendung einer Zusammensetzung gemäß einem der Ansprüche 1 bis 9, **dadurch gekennzeichnet, dass** die HBsAg oder ein Fragment davon kodierende Nukleinsäure in einem Vektor unter der Kontrolle eines zur Expression von HBsAg in einer Säugerzelle geeigneten Promotors vorliegt.

11. Verwendung einer Zusammensetzung gemäß Anspruch 10, **dadurch gekennzeichnet, dass** der Vektor ausgewählt ist aus: Plasmiden, Adenoviren, Vacciniaviren, Baculoviren, Masernviren und Retroviren.

12. Verwendung einer Zusammensetzung gemäß Anspruch 11, **dadurch gekennzeichnet, dass** der Promotor ausgewählt ist aus konstitutiven und induzierbaren Promotoren.

13. Verwendung einer Zusammensetzung gemäß einem der Ansprüche 1 bis 12, **dadurch gekennzeichnet, dass** die HBV-Infektion eine chronisch persistierende Hepatitis B ist.

14. Verwendung einer Zusammensetzung gemäß einem der Ansprüche 1 bis 12, **dadurch gekennzeichnet, dass** die HBV-vermittelte Erkrankung eine Leberzirrhose oder ein primäres Leberzellkarzinom ist.

15. Verwendung einer Zusammensetzung gemäß einem der Ansprüche 1 bis 14 **dadurch gekennzeichnet, dass** die Zusammensetzung intramuskulär, subkutan, intradermal, intravenös, mucosal oder oral verabreicht wird.

16. Verfahren zur Herstellung eines Arzneimittels zur therapeutischen Behandlung von Hepatitis B, umfassend:
a) Bestimmen des HBV-Genotyps, mit dem der Patient infiziert ist; und
b) Bereitstellen eines Arzneimittels, das wenigstens ein HBsAg eines HBV Genotyps, wobei das HBsAg aus dem S Protein (kleines HBV Oberflächenantigen) oder aus dem S Protein und der Prä-S1-Proteindomäne besteht, enthält, zur Behandlung des Patienten, dessen HBV-Genotyp im Verfahrensschritt a) bestimmt worden ist,
**dadurch gekennzeichnet, dass** sich der Genotyp des im Arzneimittel enthaltenen HBsAgs in einem T-Zellepitop vom nach a) bestimmten HBV Genotyp des Patienten unterscheidet.

17. Verfahren nach Anspruch 16, **dadurch gekennzeichnet, dass** der Genotyp durch PCR-Verfahren bestimmt wird.

18. Verwendung von wenigstens einem HBsAg eines HBV Genotyps, wobei das HBsAg aus dem S Protein (kleines HBV Oberflächenantigen) oder aus dem S Protein und der Prä-S1-Proteindomäne besteht, zur Herstellung eines Arzneimittels zur Behandlung eines Patienten, der mit einem HBV eines Genotyps infiziert ist, der sich vom HBV Genotyp des HBsAg's in einem T-Zellepitop unterscheidet, wobei der HBV Genotyp des Patienten vor der Behandlung bestimmt worden ist.

## Claims

1. Use of a composition which comprises at least two hepatitis B virus surface antigens (HBsAgs), fragments thereof which contain a T cell epitope, wherein the fragments of the at least two HBsAgs have at least 10 amino acids in common, but differ from one another at least by one amino acid, and/or nucleic acids coding for these, wherein the HBsAgs consist of the S protein (small HBV surface antigen) and wherein the HBsAgs differ in the hepatitis B virus (HBV) genotype in the S region of HBsAg, and wherein the composition comprises no HBV core antigen (HBcAg) or nucleic acid coding for this, for preparing a medicament for therapeutic treatment of an HBV infection or a disease mediated by HBV.

2. Use of a composition according to claim 1, **characterised in that** the composition comprises at least two HBsAgs and/or at least two fragments thereof.

3. Use of a composition according to claim 1 or 2, **characterised in that** the fragment/fragments of HBsAg contains/contain at least 20, in particular at least 50 amino acids.

4. Use of a composition according to claim 3, **characterised in that** the fragment includes the "a determinant" of HBsAg.

5. Use of a composition according to one of claims 1 to 4, **characterised in that** the first and the second fragment have at least 20 amino acids in common, but differ from one another at least by one amino acid.

6. Use of a composition according to claim 1, **characterised in that** the composition comprises nucleic acids coding for at least two HBsAgs or fragments thereof.

7. Use of a composition according to one of claims 1 to 6, **characterised in that** the genotype is selected from: A, B, C, D, E, F, G or H.

8. Use of a composition according to one of claims 1 to 7, **characterised in that** the composition comprises at least 3, preferably 5 different HBsAgs, fragments thereof and/or nucleic acids coding for these.

9. Use of a composition according to one of claims 1 to 8, **characterised in that** the composition comprises HBsAgs of genotypes A, B, C, D, E, F, G and H, fragments thereof and/or nucleic acids coding for these.

10. Use of a composition according to one of claims 1 to 9, **characterised in that** the nucleic acid coding for HBsAg or a fragment thereof is present in a vector under the control of a promoter suitable for expression of HBsAg in a mammalian cell.

11. Use of a composition according to claim 10, **characterised in that** the vector is selected from: plasmids, adenoviruses, vaccinia viruses, baculoviruses, measles viruses and retroviruses.

12. Use of a composition according to claim 11, **characterised in that** the promoter is selected from constitutive and inducible promoters.

13. Use of a composition according to one of claims 1 to 12, **characterised in that** the HBV infection is a chronically persistent hepatitis B.

14. Use of a composition according to one of claims 1 to 12, **characterised in that** the HBV-mediated disease is a liver cirrhosis or a primary liver cell carcinoma.

15. Use of a composition according to one of claims 1 to 14, **characterised in that** the composition is administered intramuscularly, subcutaneously, intradermally, intravenously, mucosally or orally.

16. Method of preparing a medicament for therapeutic treatment of hepatitis B, comprising:
a) determining the HBV genotype with which the patient is infected; and
b) providing a medicament which comprises at least one HBsAg of an HBV genotype, wherein the HBsAg consists of the S protein (small HBV surface antigen) or the S protein and the pre-S1 protein domain, for treatment of the patient whose HBV genotype has been determined in method step a),
**characterised in that** the genotype of the HBsAgs contained in the medicament differs from the patient's HBV genotype determined according to a) in a T cell epitope.

17. Method according to claim 16, **characterised in that** the genotype is determined by the PCR method.

18. Use of at least one HBsAg of an HBV genotype, wherein the HBsAg consists of the S protein (small HBV surface antigen) or the S protein and the pre-S 1 protein domain, for preparing a medicament for treatment of a patient who is infected with an HBV of a genotype which differs from the HBV genotype of the HBsAgs in a T cell epitope, wherein the HBV genotype of the patient has been determined before the treatment.

## Revendications

1. Utilisation d'une composition, qui comprend au moins deux antigènes de surface du virus de l'hépatite B (HBsAg), fragments de ceux-ci, contenant un épitope de cellule T, dans laquelle les fragments des au moins deux HBsAg ont au moins 10 acides aminés en commun, se distinguent l'un de l'autre au moins par un acide aminé, et/ou des acides nucléiques codant ceux-ci, dans laquelle les HBsAg se composent de la protéine S (petit antigène de surface du VHB) et dans laquelle les HBsAg dans le génotype du virus de l'hépatite B (VHB) se distinguent au niveau de la région S du HBsAg, et dans laquelle la composition ne comprend pas d'antigène nucléocapsidique du VHB (HBcAg) ou l'acide nucléique codant celui-ci, pour la préparation d'un médicament destiné au traitement thérapeutique d'une infection au VHB ou d'une affection induite par le VHB.

2. Utilisation d'une composition selon la revendication 1, **caractérisée en ce que** la composition comprend au moins deux HBsAg et/ou au moins deux fragments de ceux-ci.

3. Utilisation de la composition selon la revendication 1 ou 2, **caractérisée en ce que** le ou les fragments de HBsAg comprennent au moins 20, en particulier au moins 50 acides aminés.

4. Utilisation d'une composition selon la revendication 3, **caractérisée en ce que** le fragment comprend les « déterminants a » de HBsAg.

5. Utilisation d'une composition selon l'une des revendications 1 à 4, **caractérisée en ce que** le premier et le deuxième fragment ont au moins 20 acides aminés en commun, mais se distinguent l'un de l'autre au moins par un acide aminé.

6. Utilisation d'une composition selon la revendication 1, **caractérisée en ce que** la composition comprend au moins deux acides nucléiques codant HBsAg ou des fragments de celui-ci.

7. Utilisation d'une composition selon l'une des revendications 1 à 6, **caractérisée en ce que** le génotype est choisi parmi : A, B, C, D, E, F, G ou H.

8. Utilisation d'une composition selon l'une des revendications 1 à 7, **caractérisée en ce que** la composition comprend au moins 3, de préférence 5 HBsAg différents, fragments de ceux-ci et/ou acides nucléiques les codant.

9. Utilisation d'une composition selon l'une des revendications 1 à 8, **caractérisée en ce que** la composition comprend le HBsAg des génotypes A, B, C, D, E, F, G et H, des fragments de ceux-ci et/ou des acides nucléiques les codant.

10. Utilisation d'une composition selon l'une des revendications 1 à 9, **caractérisée en ce que** l'acide nucléique codant le HBsAg ou un fragment de celui-ci est présent dans un vecteur sous le contrôle d'un promoteur approprié pour l'expression du HBsAg dans une cellule de mammifère.

11. Utilisation d'une composition selon la revendication 10, **caractérisée en ce que** le vecteur est choisi parmi : les plasmides, les adénovirus, les virus de la vaccine, les baculovirus, les virus de la rougeole et les rétrovirus.

12. Utilisation d'une composition selon la revendication 11, **caractérisée en ce que** le promoteur est choisi parmi les promoteurs constitutifs et inductibles.

13. Utilisation d'une composition selon l'une des revendications 1 à 12, **caractérisée en ce que** l'infection au VHB est une hépatite B persistante chronique.

14. Utilisation d'une composition selon l'une des revendications 1 à 12, **caractérisée en ce que** l'affection induite par le VHB est une cirrhose du foie ou un carcinome hépatocellulaire primaire.

15. Utilisation d'une composition selon l'une des revendications 1 à 14, **caractérisée en ce que** la composition est administrée par voie intramusculaire, sous-cutanée, intradermique, intraveineuse, mucosale ou orale.

16. Procédé de préparation d'un médicament destiné au traitement thérapeutique de l'hépatite B, comprenant les étapes consistant à :
a) déterminer le génotype du VHB, avec lequel le patient est infecté ; et
b) mettre à disposition un médicament, qui contient au moins un HBsAg d'un génotype du VHB, le HBsAg se composant de la protéine S (petit antigène de surface du VHB) ou de la protéine S et du domaine pré-S1 de la protéine, pour le traitement du patient dont le génotype du VHB a été déterminé dans l'étape de procédé a),
**caractérisé en ce que** le génotype du HBsAg contenu dans le médicament se distingue du génotype de VHB déterminé à l'étape a) chez le patient au niveau d'un épitope de cellule T.

17. Procédé selon la revendication 16, **caractérisé en ce que** le génotype est déterminé par procédé PCR.

18. Utilisation d'au moins un HBsAg d'un génotype du VHB, dans laquelle le HBsAg se compose de la protéine S (petit antigène de surface du VHB) ou de la protéine S et du domaine pré-S1 de la protéine, pour la préparation d'un médicament destiné au traitement d'un patient qui est infecté avec un VHB d'un génotype qui se distingue du génotype du VHB du HBsAg au niveau d'un épitope de cellule T, le génotype VHB du patient ayant été déterminé avant le traitement.
